# EUROPÄISCHE PATENTSCHRIFT

(11) **EP 1 576 025 B1**
(45) Veröffentlichungstag und Bekanntmachung des Hinweises auf die Patenterteilung: **20.02.2013**
(21) Anmeldenummer: 03785844.6
(22) Anmeldetag: 16.12.2003
(51) Int. Cl.: C08G 18/61, C08G 18/67, A61K 8/87

(54) **ALLYLGRUPPEN-HALTIGES POLYETHERURETHAN**
POLYETHER URETHANE CONTAINING ALLYL GROUPS
POLYUR THANNE CONTENANT DES GROUPES ALLYLE

(30) Priorität: 17.12.2002 DE 10259036
(43) Veröffentlichungstag der Anmeldung: 21.09.2005
(73) Patentinhaber: BASF SE, 67056 Ludwigshafen (DE)
(72) Erfinder: NGUYEN-KIM, Son, 69502 Hemsbach (DE); MATHAUER, Klemens, 69115 Heidelberg (DE); WOOD, Claudia, 69469 Weinheim (DE)
(74) Vertreter: Gittinger, Andreas
(86) Internationale Anmeldenummer: PCT/EP2003/014357
(87) Internationale Veröffentlichungsnummer: WO 2004/055088

(56) Entgegenhaltungen:
- WO-A-01/85821
- US-A- 5 089 586
- US-A- 6 069 217

## Beschreibung

Die vorliegende Erfindung betrifft ein Mono- und/oder Polyallylpolyetherurethan, wasserlösliche oder wasserdispergierbare Polymere, die ein solches Polyetherurethan einpolymerisiert enthalten sowie kosmetische oder pharmazeutische Mittel, die ein wasserlösliches oder wasserdispergierbares Polymer auf Basis eines Mono - und/oder Polyallylpolyetherurethans enthalten.

Kosmetisch und pharmazeutisch akzeptable wasserlösliche oder wasserdispergierbare Polymere finden in Kosmetik und Medizin verbreitet Anwendung. Sie dienen beispielsweise ganz allgemein als Verdicker für diverse Arten von Formulierungen, wie z. B. Gele, Cremes oder Emulsionen. Häufig werden für diese Anwendungen verzweigte oder vernetzte wasserlösliche Polymere mit anionischen Funktionalitäten, wie z. B. vernetzte Polyacrylsäure, eingesetzt. Speziell für die Haarkosmetik werden vernetzte Polymere mit filmbildenden Eigenschaften als Conditioner dazu eingesetzt, um die Trocken- und Nasskämmbarkeit, das Anfassgefühl, den Glanz und/oder die Erscheinungsform des Haars zu verbessern sowie dem Haar antistatische Eigenschaften zu verleihen. Neben den zuvor genannten Carboxylatgruppen-haltigen Polymeren werden als Conditioner häufig vernetzte Polymere mit kationischen Funktionalitäten eingesetzt, die eine hohe Affinität zur strukturell bedingt negativ geladenen Oberfläche des Haars aufweisen. Dazu zählen beispielsweise vernetzte Copolymere aus N-Vinylpyrrolidon, quaterniertem N-Vinylimidazol, Acrylamid und Diallyldimethylammoniumchlorid (DADMAC).

Schwierigkeiten bereitet oft die Bereitstellung von Produkten mit einem komplexen Eigenschaftsprofil. So ist ein aktueller Anspruch, der an kosmetische und pharmazeutische Mittel gestellt wird, den Anteil an nicht wirkaktiven Substanzen, wie Verdickern, möglichst gering zu halten. So besteht ein Bedarf an Polymeren für kosmetische und pharmazeutische Mittel, die zum einen gute filmbildende Eigenschaften aufweisen und die zudem geeignet sind, die Rheologie der Formulierungen in der gewünschten Weise zu beeinflussen (so genannte selbstverdickende Haarpolymere). Zudem werden an kosmetische und pharmazeutische Produkte vom Verbraucher zunehmend ästhetische Anforderungen gestellt. So wird bei derartigen Produkten derzeit eine Bevorzugung von klaren, opaken Formulierungen in Form von Gelen beobachtet. Dabei zeigt sich jedoch, dass die aus dem Stand der Technik bekannten kosmetisch und pharmazeutisch akzeptablen Polymere auf Basis niedermolekularer Vernetzer in der Regel in Bezug auf mindestens eine anwendungstechnische Eigenschaft verbesserungswürdig sind. So zeichnen sie sich entweder durch eine nicht ausreichende Wasserlöslichkeit, nicht ausreichende Filmbildungseigenschaften oder die fehlende Eignung zur Bildung klarer Gele aus. Es besteht daher ein Bedarf an kosmetisch und pharmazeutisch verträglichen vorzugsweise verzweigten oder vernetzten wasserlöslichen Polymeren, die diese Nachteile vermeiden.

Die WO 99/58100 beschreibt ein kosmetisches Mittel, enthaltend wenigstens ein vernetztes, wasserlösliches oder wasserdispergierbares Polyurethan aus mindestens einem Polyurethanpräpolymer mit endständigen Isocyanatgruppen und mindestens einem Polymerisat mit gegenüber Isocyanatgruppen reaktiven Gruppen, wobei wenigstens eine der Komponenten eine Siloxangruppe enthält.

Der vorliegenden Erfindung liegt die Aufgabe zu Grunde, neue wasserlösliche oder wasserdispergierbare Polymerbausteine zur Herstellung ebenfalls wasserlöslicher oder wasserdispergierbarer kosmetisch und/oder pharmazeutisch akzeptabler Polymere zur Verfügung zu stellen. Die mit diesen Polymerbausteinen erhaltenen Polymere sollen vorzugsweise eine gute Wasserlöslichkeit und gute Filmbildungseigenschaften aufweisen und sich zur Herstellung von Produkten in Form von Gelen eignen.

Überraschenderweise wurde nun gefunden, dass diese Aufgabe durch Polyetherurethane gelöst wird, die wenigstens eine endständige Allylgruppe am Polymergerüst aufweisen.

Gegenstand der Erfindung ist daher ein Polyetherurethan, enthaltend wenigstens eine Allylgruppe, das
a) wenigstens einen Polyether, der eine gegenüber Isocyanatgruppen reaktive Gruppe und eine Allylgruppe enthält,
b) wenigstens eine Verbindung, die wenigstens zwei gegenüber Isocyanatgruppen reaktive Gruppen enthält, die ausgewählt ist unter Verbindungen mit einem zahlenmittleren Molekulargewicht von mehr als 280, die mindestens zwei aktive Wasserstoffatome und mindestens eine Siloxangruppe pro Molekül enthalten, und
c) wenigstens ein Polyisocyanat,
eingebaut enthält.

Im Rahmen der vorliegenden Erfindung umfasst der Ausdruck Alkyl geradkettige und verzweigte Alkylgruppen. Geeignete kurzkettige Alkylgruppen sind z.B. geradkettige oder verzweigte C₁-C₈-Alkyl-, bevorzugt C₁-C₆-Alkyl- und besonders bevorzugt C₁-C₄-Alkylgruppen. Dazu zählen insbesondere Methyl, Ethyl, Propyl, Isopropyl, n-Butyl, 2-Butyl, sec.-Butyl, tert.-Butyl, n-Pentyl, 2-Pentyl, 2-Methylbutyl, 3-Methylbutyl, 1,2-Dimethylpropyl, 1,1-Dimethylpropyl, 2,2-Dimethylpropyl, 1-Ethylpropyl, n-Hexyl, 2-Hexyl, 2-Methylpentyl, 3-Methylpentyl, 4-Methylpentyl, 1,2-Dimethylbutyl, 1,3-Dimethylbutyl, 2,3-Dimethylbutyl, 1,1-Dimethylbutyl, 2,2-Dimethylbutyl, 3,3-Dimethylbutyl, 1,1,2-Trimethylpropyl, 1,2,2-Trimethylpropyl, 1-Ethylbutyl, 2-Ethylbutyl, 1-Ethyl- 2-methylpropyl, n-Heptyl, 2-Heptyl, 3-Heptyl, 2-Ethylpentyl, 1-Propylbutyl, Octyl etc.

Geeignete längerkettige C₈-C₃₀-Alkyl- bzw. C₈-C₃₀-Alkenylgruppen sind geradkettige und verzweigte Alkyl- bzw. Alkenylgruppen. Bevorzugt handelt es sich dabei um überwiegend lineare Alkylreste, wie sie auch in natürlichen oder synthetischen Fettsäuren und Fettalkoholen sowie Oxoalkoholen vorkommen, die gegebenenfalls zusätzlich einfach, zweifach oder mehrfach ungesättigt sein können. Dazu zählen z.B. n-Hexyl(en), n-Heptyl(en), n-Octyl(en), n-Nonyl(en), n-Decyl(en), n-Undecyl(en), n-Dodecyl(en), n-Tridecyl(en), n-Tetradecyl(en), n-Pentadecyl(en), n-Hexadecyl(en), n-Heptadecyl(en), n-Octadecyl(en), n-Nonadecyl(en) etc.

Cycloalkyl steht vorzugsweise für C₅-C₈-Cycloalkyl, wie Cyclopentyl, Cyclohexyl, Cycloheptyl oder Cyclooctyl.

Aryl umfasst unsubstituierte und substituierte Arylgruppen und steht vorzugsweise für Phenyl, Tolyl, Xylyl, Mesityl, Naphthyl, Fluorenyl, Anthracenyl, Phenanthrenyl, Naphthacenyl und insbesondere für Phenyl, Tolyl, Xylyl oder Mesityl.

Eine geeignete Ausführungsform der erfindungsgemäßen Polyetherurethane sind Monoallylpolyetherurethane, die eine über eine Polyetherkette an das Polymergerüst gebundene Allylgruppe (-CH₂-CH=CH₂) aufweisen. Eine weitere geeignete Ausführungsform sind Polyallylpolyetherurethane, die wenigstens zwei über eine Polyetherkette an das Polymergerüst gebundene Allylgruppen aufweisen. Darunter werden auch Gemische von Verbindungen mit unterschiedlichem Allylgruppengehalt verstanden, z. B. Gemische, die wenigstens ein Mono- und wenigstens ein Polyallylpolyetherurethan enthalten.

Im Folgenden werden Verbindungen, die sich von Acrylsäure und Methacrylsäure ableiten können teilweise verkürzt durch Einfügen der Silbe "(meth)" in die von der Acrylsäure abgeleitete Verbindung bezeichnet.

Polymere auf Basis der erfindungsgemäßen Polyallylpolyetherurethane lassen sich unter Normalbedingungen (20 °C) vorteilhaft als Gele formulieren. "Gelförmige Konsistenz" zeigen Mittel, die eine höhere Viskosität als eine Flüssigkeit aufweisen und die selbsttragend sind, d. h. die eine ihnen verliehene Form ohne formstabilisierende Umhüllung behalten. Im Gegensatz zu festen Formulierungen lassen sich gelförmige Formulierungen jedoch leicht unter Anwendung von Scherkräften deformieren. Die Viskosität der gelförmigen Mittel liegen vorzugsweise in einem Bereich von größer als 600 bis etwa 60000 mPas. Vorzugsweise handelt es sich bei den Gelen um Haargele, wobei dies eine Viskosität von vorzugsweise 6000 bis 30000 mPas aufweisen.

Unter wasserlöslichen Monomeren und Polymeren werden im Rahmen der vorliegenden Erfindung Monomere und Polymere verstanden, die sich zu mindestens 1 g/l bei 20 °C in Wasser lösen. Hydrophile Monomere sind wasserlöslich oder zumindest wasserdispergierbar. Unter wasserdispergierbaren Polymeren werden Polymere verstanden, die unter Anwendung von Scherkräften beispielsweise durch Rühren in dispergierbare Partikel zerfallen. Vernetzte Copolymere auf Basis der erfindungsgemäßen Polyallylpolyetherurethane sind vorzugsweise wasserlöslich.

Das erfindungsgemäße Polyallylpolyetherurethan enthält als Komponente a) wenigstens einen Polyalkylenglykolmonoallylether (Ether des Allylalkohols mit einem Polyetherdiol). Als Komponente a) geeignete Polyalkylenglykolmonoallylether weisen im Allgemeinen ein zahlenmittleres Molekulargewicht im Bereich von etwa 150 bis 10000, bevorzugt 300 bis 5000, besonders bevorzugt 500 bis 4000, auf. Der Polyalkylenglykolrest kann sich beispielsweise von Polyethylenglykolen, Polypropylenglykolen, Polytetrahydrofuranen und Alkylenoxidcopolymeren ableiten. Geeignete Verfahren zur Herstellung von Verbindungen der Komponente a), beispielsweise ausgehend von Allylalkohol, Allylhalogeniden oder Allylglycidylether sind dem Fachmann bekannt. Derartige Produkte sind kommerziell beispielsweise von der Firma BASF Aktiengesellschaft unter der Bezeichnung Pluriol® A 010R und Pluriol® A 011R erhältlich. Bevorzugt werden als Komponente a) Allylalkoholalkoxilate eingesetzt, die vorzugsweise wenigstens ein Alkylenoxid, ausgewählt unter Ethylenoxid, Propylenoxid, Epichlorhydrin, 1,2- und 2,3-Butylenoxid einpolymerisiert enthalten. Die Allylalkoholalkoxilate können unterschiedliche Alkylenoxideinheiten statistisch verteilt oder in Form von Blöcken einpolymerisiert enthalten. Bevorzugt sind Allylalkoholethoxilate oder Copolymerisate, die Ethylenoxid enthalten, insbesondere Copolymerisate aus Ethylenoxid und Propylenoxid.

Bevorzugt ist die Komponente a) ausgewählt unter Verbindungen der allgemeinen Formel IV

CH₂=CH-CH₂-O-(CH₂CH₂O)ₘ(CH₂CH(CH₃)O)ₙ-H (IV)

worin
die Reihenfolge der Alkylenoxideinheiten beliebig ist und m und n für eine ganze Zahl von 0 bis 500 stehen, wobei die Summe aus m und n > 1 ist.

Die Verbindungen b) sind vorzugsweise ausgewählt unter
- Polysiloxanen der allgemeinen Formel I.1 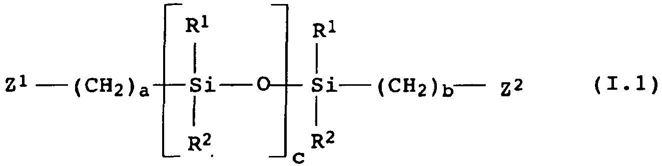 worin
   - a und b: unabhängig voneinander für 1 bis 8 stehen,
   - c: für 2 bis 100 steht,
   - R¹ und R²: unabhängig voneinander für C₁-C₈-Alkyl, Benzyl oder Phenyl stehen,
   - Z¹ und Z²: unabhängig voneinander für OH, NHR³ oder einen Rest der Formel II

   -(CH₂CH₂O)ᵥ(CH₂CH(CH₃)O)_{w}-H (II)
   stehen, wobei
   in der Formel II die Reihenfolge der Alkylenoxideinheiten beliebig ist und 5
   v und w unabhängig voneinander für eine ganze Zahl von 0 bis 200 stehen, wobei die Summe aus v und w > 0 ist,
   R³ für Wasserstoff, C₁-C₈-Alkyl oder C₅-C₈-Cycloalkyl steht;
- Polysiloxanen der allgemeinen Formel 1.2 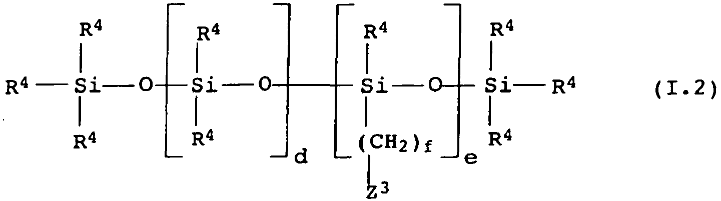 worin
   die Reihenfolge der Siloxaneinheiten beliebig ist,
   die Reste R⁴ jeweils unabhängig voneinander für C₁-C₈-Alkyl, vorzugsweise Methyl, Benzyl oder Phenyl stehen,
   d für eine ganze Zahl von 5 bis 1000 steht,
   e für eine ganze Zahl von 2 bis 100 steht,
   f für eine ganze Zahl von 2 bis 8 steht,
   Z³ für OH, NHR³, wobei R³ wie zuvor definiert ist, oder einen Rest der Formel III

   -(OCH₂CH₂)ₓ(OCH(CH₃)CH₂)_{y}-OH

   steht, wobei
in der Formel III die Reihenfolge der Alkylenoxideinheiten beliebig ist,
x und y unabhängig voneinander für eine ganze Zahl von 0 bis 200 stehen, wobei die Summe aus x und y > 0 ist,
und Mischungen davon.

Nach einer geeigneten Ausführungsform weisen die Polysiloxane b) der allgemeinen Formel I.1 keine Alkylenoxidreste der allgemeinen Formel II auf. Diese Polysiloxane b3) weisen dann vorzugsweise ein zahlenmittleres Molekulargewicht im Bereich von etwa 300 bis 5000, bevorzugt 400 bis 3000 auf.

Geeignete Polysiloxane b), die keine Alkylenoxidreste aufweisen sind z. B. die Tegomer^{®}-Marken der Fa. Goldschmidt.

Nach einer weiteren geeigneten Ausführungsform handelt es sich bei den Polysiloxanen b) um Silicon-poly(alkylenoxid)-Copolymere der Formel I.1, wobei wenigstens einer oder beide Reste Z¹ und/oder Z² für einen Rest der allgemeinen Formel II stehen.

Vorzugsweise ist in der Formel II die Summe aus v und w so gewählt, daß das Molekulargewicht der Polysiloxane b) in einem Bereich von etwa 300 bis 30000 liegt.

Bevorzugt liegt die Gesamtzahl der Alkylenoxideinheiten der Polysiloxane b), das heißt die Summe aus v und w in der Formel II in einem Bereich von etwa 3 bis 200, bevorzugt 5 bis 180.

Nach einer weiteren geeigneten Ausführungsform handelt es sich bei den Polysiloxanen b) um Silicon-poly(alkylenoxid)-Copolymere der Formel I.2, die wenigstens zwei Reste Z³ der allgemeinen Formel III aufweisen.

Vorzugsweise ist dann in der Formel III die Summe aus x und y so gewählt, dass das Molekulargewicht der Polysiloxane b) dann in einem Bereich von etwa 300 bis 30000 liegt. Bevorzugt liegt die Gesamtzahl der Alkylenoxideinheiten der Polysiloxane b), das heißt die Summe aus x und y in der Formel III in einem Bereich von etwa 3 bis 200, bevorzugt 5 bis 180.

Geeignete Silicon-poly(alkylenoxid)-Copolymere b), die z. B. unter dem internationalen Freinamen Dimethicon bekannt sind, sind die Tegopren^{®}-Marken der Fa. Goldschmidt, Belsil^{®} 6031 und 6032 der Fa. Wacker, Silvet^{®} L der Fa. Witco und Pluriol^{®} ST 4005 der Fa. BASF Aktiengesellschaft.

Geeignete Polysiloxane b) sind auch die in der EP-A-277 816 beschriebenen Polydimethylsiloxane.

Geeignete Polyisocyanate c) sind ausgewählt unter Verbindungen mit 2 bis 5 Isocyanatgruppen, Isocyanatpräpolymeren mit einer mittleren Anzahl von 2 bis 5 Isocyanatgruppen, und Mischungen davon. Dazu zählen z.B. aliphatische, cycloaliphatische und aromatische Di-, Tri- und Polyisocyanate. Geeignete Diisocyanate sind z. B. Tetramethylendiisocyanat, Hexamethylendiisocyanat, 2,3,3-Trimethylhexamethylendiisocyanat, 1,4-Cyclohexylendiisocyanat, Isophorondiisocyanat, 1,4-Phenylendiisocyanat, 2,4- und 2,6-Toluylendiisocyanat und deren Isomerengemische (z. B. 80 % 2,4- und 20 % 2,6-Isomer), 1,5-Naphthylendiisocyanat, 2,4- und 4,4'-Diphenylmethandiisocyanat. Ein geeignetes Triisocyanat ist z.B. Triphenylmethan-4,4',4"-triisocyanat. Weiterhin geeignet sind Isocyanatpräpolymere und Polyisocyanate, die durch Addition der zuvor genannten Isocyanate an polyfunktionelle hydroxyl- oder amingruppenhaltige Verbindungen erhältlich sind. Weiterhin geeignet sind Polyisocyanate, die durch Biuret- oder Isocyanuratbildung entstehen. Bevorzugt werden aliphatische und/oder cycloaliphatische Diisocyanate und insbesondere Hexamethylendiisocyanat, Isophorondiisocyanat und Mischungen davon eingesetzt.

Die Herstellung der Polyallylpolyetherurethane erfolgt durch Umsetzung der Verbindungen der Komponenten a) und, falls vorhanden, b) mit der Komponente c). Die Temperatur liegt dabei in einem Bereich von etwa 30 bis 140 °C, bevorzugt etwa 40 bis 100 °C. Die Reaktion kann ohne Lösungsmittel oder in einem geeigneten inerten Lösungsmittel oder Lösungsmittelgemisch erfolgen. Geeignete Lösungsmittel sind aprotisch polare Lösungsmittel, z. B. Tetrahydrofuran, Essigsäureethylester, N-Methylpyrrolidon, Dimethylformamid und bevorzugt Ketone, wie Aceton und Methylethylketon. Vorzugsweise erfolgt die Reaktion unter einer Inertgasatmosphäre, wie z. B. unter Stickstoff. Des Weiteren erfolgt die Reaktion vorzugsweise bei Umgebungsdruck oder unter erhöhtem Druck.

Die Umsetzung der Komponenten erfolgt so, dass die erfindungsgemäßen Polyetherurethane wenigstens eine bzw. für Polyallylpolyetherurethane wenigstens zwei endständige Allylgruppen aufweisen. Beim Einsatz von Verbindungen der Komponente b) ist es zweckmäßig, zunächst aus den Verbindungen a) und c) ein NCO-gruppenhaltiges Präpolymer herzustellen und dieses abschließend mit den Verbindungen b) umzusetzen.

Vorteilhafterweise ist es möglich, bei entsprechender Verfahrensführung bei der Herstellung der erfindungsgemäßen Polyetherurethane auf den Einsatz organischer und insbesondere organischer nicht wassermischbarer Lösungsmittel zu verzichten oder deren Einsatzmenge deutlich zu verringern. Dies ist von Vorteil, da für viele kosmetische und pharmazeutische Anwendungen diese Lösungsmittel vor der Formulierung der Produkte entfernt werden müssen.

Ein geeignetes Verfahren zur Herstellung eines erfindungsgemäβen Polyetherurethans ist ein Verfahren, bei dem man
i) in einer ersten Stufe die Verbindungen a), gegebenenfalls einen Teil der Verbindungen b) und wenigstens einen Teil der Polyisocyanate c) ohne Zusatz eines Lösungsmittels, bei einer Temperatur von mindestens 60 °C und bei einem Verhältnis von Isocyanatgruppen-Äquivalenten zu Äquivalenten von gegenüber Isocyanatgruppen reaktiven Gruppen in einem Bereich von 1,5:1 bis 2,2:1, zu einem isocyanatgruppenhaltigen Prepolymer umsetzt, und
ii) in einer zweiten Stufe das in Schritt i) erhaltene Prepolymer mit den nicht bereits in Schritt i) eingesetzten Verbindungen b) und c) zu dem Polyetherurethan umsetzt.

Bevorzugt wird in der Stufe i) ein Prepolymer mit einer Glasübergangstemperatur T_{G} von höchstens 100 °C, bevorzugt von höchstens 60 °C, erhalten.

Vorzugsweise erfolgt die Umsetzung in Schritt i) bei einer Temperatur, die höher ist als die Glasübergangstemperatur des Prepolymers.

In einer ersten Ausführungsform weisen die in Schritt ii) eingesetzten Verbindungen b) Hydroxylgruppen als gegenüber Isocyanatgruppen reaktive Gruppen auf, und die Umsetzung erfolgt ohne Zusatz eines Lösungsmittels.

In einer zweiten Ausführungsform weisen die in Schritt ii) eingesetzten Verbindungen b) primäre oder sekundäre Aminogruppen als gegenüber Isocyanatgruppen reaktive Gruppen auf, und die Umsetzung erfolgt in Gegenwart eines protisch-polaren Lösungsmittels.

Die Komponenten werden bevorzugt in solchen Mengen eingesetzt, dass das resultierende Allylpolyetherurethan im Wesentlichen keine freien NCO-Gruppen aufweist. Das Verhältnis von NCO-Äquivalenten der Verbindungen der Komponente c) zu Äquivalenten aktiver Wasserstoffatome der Komponenten a) und, falls vorhanden, b) liegt vorzugsweise in einem Bereich von etwa 1:1,01 bis 1:3, bevorzugt 1:1,1 bis 1:2,5, insbesondere 1:1,2 bis 1:2,2. Gegebenenfalls noch vorhandene freie Isocyanatgruppen der Allylpolyetherurethane können durch anschließende Umsetzung mit Aminen, vorzugsweise Aminoalkoholen, oder mit C₂-C₄-Alkoholen, wie Ethanol, n-Propanol oder Isopropanol inaktiviert werden. Geeignete Amine und Aminoalkohole sind die zuvor als Komponente b1) genannten, bevorzugt 2-Amino-2-methyl-1-propanol.

Die erfindungsgemäßen Allylpolyetherurethane weisen vorzugsweise 1 bis 40, besonders bevorzugt 2 bis 25 Allylgruppen auf. Eine spezielle Ausführungsform sind Monoallylpolyetherurethane. Eine weitere spezielle Ausführungsform sind Polyallylpolyetherurethane, die zwei Allylgruppen aufweisen. Die zuvor genannten Ausführungsformen eignen sich vorteilhaft zur Herstellung niedrig verzweigter oder vernetzter wasserlöslicher oder wasserdispergierbarer Copolymere. Eine weitere spezielle Ausführungsform sind Polyallylpolyetherurethane, die 3 bis 40, vorzugsweise 4 bis 25 Allylgruppen aufweisen. Diese eignen sich in vorteilhafter Weise zur Herstellung etwas höher verzweigter oder vernetzter wasserlöslicher oder wasserdispergierbarer Copolymere.

Das zahlenmittlere Molekulargewicht der Allylpolyetherurethane beträgt vorzugsweise etwa 300 bis 25000, besonders bevorzugt 400 bis 10000.

Besonders bevorzugt sind Allylpolyetherurethane, die
a) 40 bis 98 Gew.-%, vorzugsweise 80 bis 96 Gew.-%, eines Allylalkoholethoxilats mit einem zahlenmittleren Molekulargewicht von 400 bis 4000 und
b) 0 bis 20 Gew.-%, vorzugsweise 1 bis 15 Gew.-%, wenigstens eines Diols mit einem Molekulargewicht im Bereich von 56 bis 280 g/mol, und
c) 2 bis 40 Gew.-%, vorzugsweise 4 bis 20 Gew.-%, wenigstens ein Diisocyanat, insbesondere Hexamethylendiisocyanat und/oder Isophorondiisocyanat,
eingebaut enthalten.

Des Weiteren besonders bevorzugt sind Allylpolyetherurethane, die
a) 50 bis 90 Gew.-% eines Allylalkoholethoxilats mit einem zahlenmittleren Molekulargewicht von 400 bis 4000
b) 1 bis 30 Gew.-% eines Polyetherpolyols mit einem zahlenmittleren Molekulargewicht im Bereich von 300 bis 5000 und/oder eines alkoxilierten Polydimethylsiloxans mit einem zahlenmittleren Molekulargewicht im Bereich von 3000 bis 20000, und
c) 2 bis 49 Gew.-%, vorzugsweise 3 bis 30 Gew.-%, wenigstens ein Diisocyanat, insbesondere Hexamethylendiisocyanat und/oder Isophorondiisocyanat,
eingebaut enthalten.

Die erfindungsgemäßen Polyetherurethane eignen sich vorteilhaft zur Herstellung kosmetisch und/oder pharmazeutisch akzeptabler wasserlöslicher (oder zumindest wasserdispergierbarer) linearer, verzweigter oder vernetzter Polymere. Die erfindungsgemäßen wasserlöslichen oder wasserdispergierbaren Polymere umfassen ganz allgemein die Produkte der radikalischen Copolymerisation erfindungsgemäßer Polyetherurethane mit Verbindungen, die wenigstens eine α,β-ethylenisch ungesättigte Doppelbindung aufweisen. Dabei kann es sich um Copolymerisate handeln, bei denen die Polyallylpolyetherurethane über eine, zwei oder, falls vorhanden, mehr als zwei ihrer Allylgruppen kovalent angebunden sind. Umfasst sind jedoch auch Copolymerisate, die noch freie Allylgruppen aufweisen, Verfahrensprodukte aus einer zumindest teilweisen Pfropfung der α,β-ethylenisch ungesättigten Verbindungen auf die Polyetherketten der Allylpolyetherurethane, Mischungen von Homo- und Copolymerisaten der ethylenisch ungesättigten Verbindungen mit den Allylpolyetherurethanen sowie beliebige Mischungen der zuvor genannten Komponenten. Polymere auf Basis der erfindungsgemäßen Allylpolyetherurethane weisen sowohl gegenüber Polymeren auf Basis niedermolekularer Vernetzer (z. B. Diacrylate, Methylenbisacrylamid, Divinylbenzol, Triallylamin, etc.) als auch gegenüber Polymeren auf Basis hochmolekularer Vernetzer mit mindestens zwei Vinylgruppen vorteilhafte Eigenschaften auf. So zeichnen sich Polymere auf Basis von Polyallylpolyetherurethanen gegenüber konventionell vernetzten Polymeren durch eine verbesserte Wasserlöslichkeit, gute rheologische (verdickende) Eigenschaften, hohe Flexibilität, gute Filmbildungseigenschaften und/oder die Befähigung zur Bildung klarer Gele aus.

Ein weiterer Gegenstand der Erfindung ist ein wasserlösliches oder wasserdispergierbares Polymer, das wenigstens ein Polyallylpolyetherurethan, wie zuvor definiert, und wenigstens eine radikalisch polymerisierbare Verbindung, die wenigstens eine α,β-ethylenisch ungesättigte Doppelbindung aufweist, einpolymerisiert enthält.

Die erfindungsgemäßen wasserlöslichen oder wasserdispergierbaren Polymere enthalten vorzugsweise 1 bis 25 Gew.-%, besonders bevorzugt 2 bis 20 Gew.-%, bezogen auf das Gesamtgewicht der zur Polymerisation eingesetzten Komponenten, wenigstens eines Polyallylpolyetherurethans einpolymerisiert.

Vorzugsweise enthalten die Polymere zusätzlich zu dem Polyallylpolyetherurethan wenigstens eine radikalisch polymerisierbare hydrophile nichtionische Verbindung M1) einpolymerisiert.

Das erfindungsgemäße wasserlösliche oder wasserdispergierbare Polymer enthält vorzugsweise 50 bis 99 Gew.-%, besonders bevorzugt 60 bis 98 Gew.-%, insbesondere 70 bis 95 Gew.-%, bezogen auf das Gesamtgewicht der zur Polymerisation eingesetzten Komponenten, wenigstens einer radikalisch polymerisierbaren nichtionischen Verbindung M1) einpolymerisiert.

Vorzugsweise ist die radikalisch polymerisierbare nichtionische Verbindung M1) ausgewählt unter primären Amiden α,β-ethylenisch ungesättigter Monocarbonsäuren, N-Vinyllactamen, N-Vinylamiden gesättigter Monocarbonsäuren, Estern α,β-ethylenisch ungesättigter Mono- und Dicarbonsäuren mit C₂-C₄-Alkandiolen, Estern und Amiden α,β-ethylenisch ungesättigter Mono- und Dicarbonsäuren mit C₂-C₄-Aminoalkoholen, die eine primäre oder sekundäre Aminogruppe aufweisen, Vinylethern, nichtionischen, hydrophilen vinyl- und allylsubstituierten heterocyclischen Verbindungen und Mischungen davon.

Als nichtionische Monomere M1) geeignete primäre Amide α,β-ethylenisch ungesättigter Monocarbonsäuren sind beispielsweise Acrylsäureamid, Methacrylsäureamid, Ethacrylsäureamid und Mischungen davon. Bevorzugt sind Acrylsäureamid und/oder Methacrylsäureamid.

Bevorzugte hydrophile nichtionische Monomere M1) sind N-Vinyllactame und deren Derivate, die z. B. einen oder mehrere C₁-C₆-Alkylsubstituenten, wie Methyl, Ethyl, n-Propyl, Isopropyl, n-Butyl, sec.-Butyl, tert.-Butyl etc. aufweisen können. Dazu zählen z. B. N-Vinylpyrrolidon, N-Vinylpiperidon, N-Vinylcaprolactam, N-Vinyl-5-methyl-2-pyrrolidon, N-Vinyl-5-ethyl-2-pyrrolidon, N-Vinyl-6-methyl-2-piperidon, N-Vinyl-6-ethyl-2-piperidon, N-Vinyl-7-methyl-2-caprolactam, N-Vinyl-7-ethyl-2-caprolactam etc. Bevorzugt werden N-Vinylpyrrolidon und/oder N-Vinylcaprolactam eingesetzt.

Als hydrophile nichtionische Monomere M1) geeignete N-Vinylamide sind beispielsweise N-Vinylformamid, N-Vinyl-N-methylformamid, N-Vinylacetamid, N-Vinyl-N-methylacetamid, N-Vinyl-N-ethylacetamid, N-Vinylpropionamid, N-Vinyl-N-methylpropionamid, N-Vinyl-butyramid und Mischungen davon. Bevorzugt wird N-Vinylformamid eingesetzt.

Geeignete hydrophile nichtionische Monomere M1) sind weiterhin die Ester α,β-ethylenisch ungesättigter Mono- und Dicarbonsäuren, wie Acrylsäure, Methacrylsäure, Fumarsäure, Maleinsäure, Itaconsäure, Crotonsäure etc., mit C₁-C₄-Alkandiolen. Dazu zählen z. B. 2-Hydroxyethylacrylat, 2-Hydroxyethylmethacrylat, 2-Hydroxyethylethacrylat, 2-Hydroxypropylacrylat, 2-Hydroxypropylmethacrylat, 3-Hydroxypropylacrylat, 3-Hydroxypropylmethacrylat, 3-Hydroxybutylacrylat, 3-Hydroxybutylmethacrylat, 4-Hydroxybutylacrylat, 4-Hydroxybutylmethacrylat, etc. Vorzugsweise werden Hydroxyethylacrylat und Hydroxyethylmethacrylat eingesetzt. Geeignete Monomere sind auch die Ester der zuvor genannten Säuren mit Triolen und Polyolen, wie z. B. Glycerin, Erythrit, Pentaerythrit, Sorbit etc.

Vorzugsweise enthalten die wasserlöslichen oder wasserdispergierbaren Polymere wenigstens eine hydrophile nichtionische Verbindung M1) einpolymerisiert, die ausgewählt ist unter Acrylsäureamid, Methacrylsäureamid, N-Vinylpyrrolidon, N-Vinylcaprolactam, N-Vinylformamid, N-Vinylacetamid und Mischungen davon.

Vorzugsweise enthalten die erfindungsgemäßen wasserlöslichen oder wasserdispergierbaren,Polymere zusätzlich zu wenigstens einem Polyallylpolyetherurethan und wenigstens einer radikalisch polymerisierbaren hydrophilen nichtionischen Verbindung M1) wenigstens eine radikalisch polymerisierbare Verbindung M2) mit einer α,β-ethylenisch ungesättigten Doppelbindung und mindestens einer ionogenen und/oder ionischen Gruppe pro Molekül einpolymerisiert.

Das erfindungsgemäße Polymer enthält vorzugsweise bis zu 25 Gew.-%, besonders bevorzugt 1 bis 20 Gew.-%, insbesondere 2 bis 15 Gew.-%, bezogen auf das Gesamtgewicht der zur Polymerisation eingesetzten Komponenten wenigstens eines Monomers M2) mit mindestens einer ionogenen und/oder ionischen Gruppe pro Molekül einpolymerisiert.

Die ionogenen bzw. ionischen Gruppen sind vorzugsweise ausgewählt unter Carbonsäuregruppen, Sulfonsäuregruppen und/oder Phosphonsäuregruppen und deren durch teilweise oder vollständige Neutralisation mit einer Base erhältlichen Salze, sowie Aminogruppen, die teilweise oder vollständig protoniert und quaternisiert sein können.

Vorzugsweise sind die Monomere M2) mit anionogenen/anionischen Gruppen ausgewählt unter monoethylenisch ungesättigten Carbonsäuren, Sulfonsäuren, Phosphonsäuren und Salzen und Mischungen davon. Dazu zählen monoethylenisch ungesättigte Mono- und Dicarbonsäuren mit 3 bis 25 vorzugsweise 3 bis 6 C-Atomen, die auch in Form ihrer Salze oder Anhydride eingesetzt werden können. Beispiele hierfür sind Acrylsäure, Methacrylsäure, Ethacrylsäure, α-Chloracrylsäure, Crotonsäure, Maleinsäure, Maleinsäureanhydrid, Itaconsäure, Citraconsäure, Mesaconsäure, Glutaconsäure, Aconitsäure und Fumarsäure. Dazu zählen weiterhin die Halbester von monoethylenisch ungesättigten Dicarbonsäuren mit 4 bis 10 vorzugsweise 4 bis 6 C-Atomen, z. B. von Maleinsäure wie Maleinsäuremonomethylester. Dazu zählen auch monoethylenisch ungesättigte Sulfonsäuren und Phosphonsäuren, beispielsweise Vinylsulfonsäure, Allylsulfonsäure, Sulfoethylacrylat, Sulfoethylmethacrylat, Sulfopropylacrylat, Sulfopropylmethacrylat, 2-Hydroxy-3-acryloxypropylsulfonsäure, 2-Hydroxy-3-methacryloxypropylsulfonsäure, Styrolsulfonsäure, 2-Acrylamido-2-methylpropansulfonsäure, Vinylphosphonsäure und Allylphosphonsäure und die Salze, insbesondere die Natrium-, Kalium- und Ammoniumsalze dieser Säuren. Die Monomere mit anionogenen/anionischen Gruppen können als solche oder als Mischungen untereinander eingesetzt werden. Die angegebenen Gewichtsanteile beziehen sich sämtlich auf die Säureform.

Vorzugsweise werden zur Herstellung der erfindungsgemäßen wasserlöslichen oder wasserdispergierbaren Polymere Monomere mit anionogenen/anionischen Gruppen eingesetzt, die ausgewählt sind unter Acrylsäure, Methacrylsäure, Ethacrylsäure, α-Chloracrylsäure, Crotonsäure, Maleinsäure, Maleinsäureanhydrid, Fumarsäure, Itaconsäure, Citraconsäure, Mesaconsäure, Glutaconsäure, Aconitsäure und Mischungen davon.

Besonders bevorzugt sind die Monomere mit anionogenen/anionischen Gruppen ausgewählt unter Acrylsäure, Methacrylsäure und Mischungen, die Acrylsäure und/oder Methacrylsäure enthalten.

Nach einer weiteren Ausführungsform handelt es sich bei den Monomeren M2) mit ionogenen/ionischen Gruppen um solche mit kationogenen/kationischen Gruppen.

Bevorzugt handelt es sich bei den kationogenen und/oder kationischen Gruppen um stickstoffhaltige Gruppen, wie primäre, sekundäre und tertiäre Aminogruppen sowie quaternäre Ammoniumgruppen. Vorzugsweise handelt es sich bei den stickstoffhaltigen Gruppen um tertiäre Aminogruppen oder quaternäre Ammoniumgruppen. Geladene kationische Gruppen lassen sich aus den Aminstickstoffen entweder durch Protonierung, z. B. mit Carbonsäuren, wie Milchsäure, oder Mineralsäuren, wie Phosphorsäure, Schwefelsäure und Salzsäure, oder durch Quaternisierung, z. B. mit Alkylierungsmitteln, wie C₁-C₄-Alkylhalogeniden oder -sulfaten, erzeugen. Beispiele solcher Alkylierungsmittel sind Ethylchlorid, Ethylbromid, Methylchlorid, Methylbromid, Dimethylsulfat und Diethylsulfat.

Geeignete Monomere sind z. B. die Ester von a,β-ethylenisch ungesättigten Mono- und Dicarbonsäuren mit Aminoalkoholen. Bevorzugte Aminoalkohole sind C₂-C₁₂-Aminoalkoholen, welche am Aminstickstoff C₁-C₈-dialkyliert sind. Als Säurekomponente dieser Ester eignen sich z. B. Acrylsäure, Methacrylsäure, Fumarsäure, Maleinsäure, Itaconsäure, Crotonsäure, Maleinsäureanhydrid, Monobutylmaleat und Gemische davon. Bevorzugt werden Acrylsäure, Methacrylsäure und deren Gemische eingesetzt. Bevorzugt sind N,N-Dimethylaminomethyl(meth)acrylat, N,N-Dimethylaminoethyl(meth)acrylat, N,N-Diethylaminoethyl(meth)acrylat, N,N-Dimethylaminopropyl(meth)acrylat, N,N-Diethylaminopropyl(meth)acrylat und N,N-Di-methylaminocyclohexyl(meth)acrylat.

Geeignete Monomere M2) sind weiterhin die Amide der zuvor genannten α,β-ethylenisch ungesättigten Mono- und Dicarbonsäuren mit Diaminen, welche mindestens eine primäre oder sekundäre Aminogruppe aufweisen. Bevorzugt sind Diamine, die eine tertiäre und ein primäre oder sekundäre Aminogruppe aufweisen. Bevorzugt werden als Monomere N-[2-(dimethylamino)ethyl]acrylamid, N-[2-(dimethylamino)ethyl]methacrylamid, N-[3-(dimethylamino)propyl]acrylamid, N-[3-(dimethylamino)propyl]methacrylamid, N-[4-(dimethylamino)butyl]acrylamid, N-[4-(dimethylamino)- butyl]methacrylamid, N-[2-(diethylamino)ethyl]acrylamid, N-[4-(dimethylamino)cyclohexyl]acrylamid, N-[4-(dimethylamino)cyclohexyl]methacrylamid etc eingesetzt. Besonders bevorzugt werden N-[3-(dimethyl-amino)propyl]acrylamid und/oder N-[3-(dimethylamino)propyl]methacrylamid eingesetzt.

Geeignete Monomere M2) sind weiterhin N,N-Diallylamine und N,N-Diallyl-N-alkylamine und deren Säureadditionssalze und Quaternisierungsprodukte. Alkyl steht dabei vorzugsweise für C₁-C₂₄-Alkyl. Bevorzugt sind N,N-Diallyl-N-methylamin und N,N-Diallyl-N,N-dimethylammonium-Verbindungen, wie z. B. die Chloride und Bromide.

Geeignete Monomere M2) mit kationogenen/kationischen Gruppen sind weiterhin vinyl- und allylsubstituierte Stickstoffheterocyclen, wie N-Vinylimidazol, N-Vinyl-2-methylimidazol, vinyl- und allylsubstituierte heteroaromatische Verbindungen, wie 2- und 4-Vinylpyridin, 2- und 4-Allylpyridin, und die Salze davon.

Die erfindungsgemäßen wasserlöslichen oder wasserdispergierbaren Polymere können gewünschtenfalls bis zu 15 Gew.-%, besonders bevorzugt 0,1 bis 10 Gew.-%, wenigstens eines weiteren Monomers M3) einpolymerisiert enthalten. Vorzugsweise sind diese zusätzlichen Monomere ausgewählt unter Estern α,β-ethylenisch ungesättigter Mono- und Dicarbonsäuren mit C₁-C₃₀-Alkanolen, N-Alkyl- und N,N-Dialkylamiden α,β-ethylenisch ungesättigter Monocarbonsäuren, Estern von Vinylalkohol und Allylalkohol mit C₁-C₃₀-Monocarbonsäuren, Vinylaromaten, Vinylhalogeniden, Vinylidenhalogeniden, C₁-C₈-Monoolefinen, nicht aromatischen Kohlenwasserstoffen mit mindestens zwei konjugierten Doppelbindungen, Siloxanmacromeren und Mischungen davon. Geeignete N-Alkyl- und N,N-Dialkylamide α,β-ethylenisch ungesättigter Monocarbonsäuren sind beispielsweise N-Methyl(meth)acrylamid, N-Ethyl(meth)acrylamid, N-Propyl(meth)acrylamid, N-tert.-Butyl(meth)acrylamid, N,N-Dimethyl(meth)acrylamid, N,N-Diethyl(meth)acrylamid, Piperidinyl(meth)acrylamid, Morpholinyl(meth)acrylamid, etc.

Die erfindungsgemäßen wasserlöslichen oder wasserdispergierbaren Polymere können gewünschtenfalls wenigstens einen Vernetzer, d. h. eine Verbindung mit zwei oder mehr als zwei ethylenisch ungesättigten Doppelbindungen einpolymerisiert enthalten. Vorzugsweise werden Vernetzer in einer Menge von 0,01 bis 10 Gew.-%, besonders bevorzugt 0,1 bis 3 Gew.-% bezogen auf das Gesamtgewicht der Komponenten zur Polymerisation verwendeten Komponenten eingesetzt.

Als vernetzende Monomere können Verbindungen mit mindestens zwei ethylenisch ungesättigten Doppelbindungen eingesetzt werden, wie zum Beispiel Ester von ethylenisch ungesättigten Carbonsäuren, wie Acrylsäure oder Methacrylsäure und mehrwertigen Alkoholen, Ether von mindestens zweiwertigen Alkoholen, wie zum Beispiel Vinylether oder Allylether.

Beispiele für die zu Grunde liegenden Alkohole sind zweiwertige Alkohole wie 1,2-Ethandiol, 1,2-Propandiol, 1,3-Propandiol, 1,2-Butandiol, 1,3-Butandiol, 2,3-Butandiol, 1,4-Butandiol, But-2-en-1,4-diol, 1,2-Pentandiol, 1,5-Pentandiol, 1,2-Hexandiol, 1,6-Hexandiol, 1,10-Decandiol, 1,2-Dodecandiol, 1,12-Dodecandiol, Neopentylglykol, 3-Methylpentan-1,5-diol, 2,5-Dimethyl-1,3-hexandiol, 2,2,4-Trimethyl-1,3-pentandiol, 1,2-Cyclohexandiol, 1,4-Cyclohexandiol, 1,4-Bis(hydroxymethyl)cyclohexan, Hydroxypivalinsäure-neopentylglycolmonoester, 2,2-Bis(4-hydroxyphenyl)-propan, 2,2-Bis[4-(2-hydroxypropyl)phenyl]propan, Diethylenglykol, Triethylenglykol, Tetraethylenglykol, Dipropylenglykol, Tripropylenglykol, Tetrapropylenglykol, 3-Thio-pentan-1,5-diol, sowie Polyethylenglykole, Polypropylenglykole und Polytetrahydrofurane mit Molekulargewichten von jeweils 200 bis 10000. Außer den Homopolymerisaten des Ethylenoxids bzw. Propylenoxids können auch Blockcopolymerisate aus Ethylenoxid oder Propylenoxid oder Copolymerisate, die Ethylenoxid- und Propylenoxid-Gruppen eingebaut enthalten, eingesetzt werden. Beispiele für zu Grunde liegende Alkohole mit mehr als zwei OH-Gruppen sind Trimethylolpropan, Glycerin, Pentaerythrit, 1,2,5-Pentantriol, 1,2,6-Hexantriol, Triethoxycyanursäure, Sorbitan, Zucker wie Saccharose, Glucose, Mannose. Selbstverständlich können die mehrwertigen Alkohole auch nach Umsetzung mit Ethylenoxid oder Propylenoxid als die entsprechenden Ethoxylate bzw. Propoxylate eingesetzt werden. Die mehrwertigen Alkohole können auch zunächst durch Umsetzung mit Epichlorhydrin in die entsprechenden Glycidylether überführt werden.

Weitere geeignete Vernetzer sind die Vinylester oder die Ester einwertiger, ungesättigter Alkohole mit ethylenisch ungesättigten C₃-C₆-Carbonsäuren, beispielsweise Acrylsäure, Methacrylsäure, Itaconsäure, Maleinsäure oder Fumarsäure. Beispiele für solche Alkohole sind Allylalkohol, 1-Buten-3-ol, 5-Hexen-1-ol, 1-Octen-3-ol, 9-Decen-1-ol, Dicyclopentenylalkohol, 10-Undecen-1-ol, Zimtalkohol, Citronellol, Crotylalkohol oder cis-9-Octadecen-1-ol. Man kann aber auch die einwertigen, ungesättigten Alkohole mit mehrwertigen Carbonsäuren verestern, beispielsweise Malonsäure, Weinsäure, Trimellitsäure, Phthalsäure, Terephthalsäure, Citronensäure oder Bernsteinsäure.

Weitere geeignete Vernetzer sind Ester ungesättigter Carbonsäuren mit den oben beschriebenen mehrwertigen Alkoholen, beispielsweise der Ölsäure, Crotonsäure, Zimtsäure oder 10-Undecensäure.

Außerdem geeignet sind geradkettige oder verzweigte, lineare oder cyclische aliphatische oder aromatische Kohlenwasserstoffe, die über mindestens zwei Doppelbindungen verfügen, welche bei den aliphatischen Kohlenwasserstoffen nicht konjugiert sein dürfen, z. B. Divinylbenzol, Divinyltoluol, 1,7-Octadien, 1,9-Decadien, 4-Vinyl-1-cyclohexen, Trivinylcyclohexan oder Polybutadiene mit Molekulargewichten von 200 bis 20000.

Ferner geeignet sind Amide von ungesättigten Carbonsäuren, wie z. B., Acryl- und Methacrylsäure, Itaconsäure, Maleinsäure, und N-Allylaminen von mindestens zweiwertigen Aminen, wie zum Beispiel 1,2-Diaminomethan, 1,2-Diaminoethan, 1,3-Diaminopropan, 1,4-Diaminobutan, 1,6-Diaminohexan, 1,12-Dodecandiamin, Piperazin, Diethylentriamin oder Isophorondiamin. Ebenfalls geeignet sind die Amide aus Allylamin und ungesättigten Carbonsäuren wie Acrylsäure, Methacrylsäure, Itaconsäure, Maleinsäure, oder mindestens zweiwertigen Carbonsäuren, wie sie oben beschrieben wurden.

Ferner sind Triallylamin oder entsprechende Ammoniumsalze, z. B. Triallylmethylammoniumchlorid oder -methylsulfat, als Vernetzer geeignet.

Weiterhin können N-Vinylverbindungen von Harnstoffderivaten, mindestens zweiwertigen Amiden, Cyanuraten oder Urethanen, beispielsweise von Harnstoff, Ethylenharnstoff, Propylenharnstoff oder Weinsäurediamid, z. B. N,N'-Divinylethylenharnstoff oder N,N'-Divinylpropylenharnstoff eingesetzt werden.

Weitere geeignete Vernetzer sind Divinyldioxan, Tetraallylsilan oder Tetravinylsilan.

Besonders bevorzugte Vernetzer sind beispielsweise Methylenbisacrylamid, Divinylbenzol, Triallylamin und Triallylammoniumsalze, Divinylimidazol, N,N'-Divinylethylenharnstoff, Umsetzungsprodukte mehrwertiger Alkohole mit Acrylsäure oder Methacrylsäure, Methacrylsäureester und Acrylsäureester von Polyalkylenoxiden oder mehrwertigen Alkoholen, die mit Ethylenoxid und/oder Propylenoxid und/oder Epichlorhydrin umgesetzt worden sind, sowie Allyl- oder Vinylether von mehrwertigen Alkoholen, beispielsweise 1,2-Ethandiol, 1,4-Butandiol, Diethylenglykol, Trimethylolpropan, Glycerin, Pentaerythrit, Sorbitan und Zucker wie Saccharose, Glucose, Mannose.

Besonders bevorzugt als Vernetzer sind Pentaerythrittriallylether, Allylether von Zuckern wie Saccharose, Glucose, Mannose, Divinylbenzol, N,N'-Methylenbisacrylamid, N,N'-Divinylethylenharnstoff, und (Meth-)Acrylsäureester von Glykol, Butandiol, Trimethylolpropan oder Glycerin oder (Meth)Acrylsäureester von mit Ethylenoxid und/oder Epichlorhydrin umgesetzten Glykol, Butandiol, Trimethylolpropan oder Glycerin. Ganz besonders bevorzugt sind N,N'-Methylenbisacrylamid, Diallylweinsäurediamid, Diallylphthalat, Diallylharnstoff, Glycoldi(meth)acrylat, Allyl(meth)acrylat sowie Polyallylether.

Nach einer geeigneten Ausführungsform erfolgt die Copolymerisation zur Herstellung der erfindungsgemäßen wasserlöslichen oder wasserdispergierbaren Polymere in Gegenwart wenigstens einer Verbindung der Komponente d), die ausgewählt ist unter
d1) polyetherhaltigen Verbindungen,
d2) Polymeren, die mindestens 50 Gew.-% Wiederholungseinheiten aufweisen, die sich von Vinylalkohol ableiten,
d3) Stärke und Stärkederivaten,
und Mischungen davon.

Erfolgt die radikalische Copolymerisation in Gegenwart wenigstens einer Verbindung der Komponente d) werden Copolymere mit vorteilhaften Eigenschaften erhalten. Dies kann beispielsweise auf eine Wirkung der Komponente d) als Schutzkolloid oder Emulgator zurückzuführen sein. Dies kann beispielsweise auch aus einer zumindest teilweisen Pfropfung auf die Komponente d) als Pfropfgrundlage resultieren. Es sind jedoch auch andere Mechanismen als eine Pfropfung vorstellbar. Die erfindungsgemäßen Copolymere umfassen ganz allgemein die Verfahrensprodukte der radikalischen Copolymerisation worunter z. B. reine Pfropfpolymerisate, Mischungen von Pfropfpolymerisaten mit ungepfropften Verbindungen der Komponente d), Copolymerisate der zuvor genannten Monomere sowie beliebige Mischungen verstanden werden. Anteile von ungepfropften Verbindungen der Komponente d) können je nach Verwendungszweck der ampholytischen Copolymere von Vorteil sein. Speziellen Verbindungen d1) kann beispielsweise eine Wirkung als Emulgator oder Schutzkolloid zukommen.

Vorzugsweise beträgt die Einsatzmenge der Komponente d) 1 bis 25 Gew.-%, besonders bevorzugt 3 bis 20 Gew.-%, bezogen auf das Gesamtgewicht der zur Polymerisation eingesetzten Komponenten.

Geeignete polyetherhaltige Verbindungen d1) sind beispielsweise wasserlösliche oder wasserdispergierbare nichtionische Polymere, die Alkylenoxid-Wiederholungseinheiten aufweisen. Vorzugsweise beträgt der Anteil an Alkylenoxid-Wiederholungseinheiten mindestens 30 Gew.-%, bezogen auf das Gesamtgewicht der Verbindung d1). Geeignete polyetherhaltige Verbindungen d1) sind beispielsweise Polyalkylenglykole, Polyester auf Basis von Polyalkylenglykolen, Polyetherurethane sowie polyalkylenoxidgruppenhaltige Silikonderivate.

Als Komponente d1) geeignete Polyalkylenglykole weisen im Allgemeinen ein zahlenmittleres Molekulargewicht im Bereich von etwa 150 bis 100000, bevorzugt 300 bis 50000, besonders bevorzugt 500 bis 40000 auf. Geeignete Polyalkylenglykole sind beispielsweise Polyethylenglykole, Polypropylenglykole, Polytetrahydrofurane und Alkylenoxidcopolymere. Geeignete Alkylenoxide zur Herstellung von Alkylenoxidcopolymeren sind z. B. Ethylenoxid, Propylenoxid, Epichlorhydrin, 1,2- und 2,3-Butylenoxid. Die Alkylenoxidcopolmere können die Alkylenoxideinheiten statistisch verteilt oder in Form Blöcken einpolymerisiert enthalten. Vorteilhafterweise verwendet man Homopolymerisate des Ethylenoxids oder Copolymerisate, die Ethylenoxid enthalten. Vorzugsweise beträgt der Anteil an von Ethylenoxid abgeleiteten Wiederholungseinheiten 40 bis 99 Gew.-%. Geeignet sind beispielsweise Copolymerisate aus Ethylenoxid und Propylenoxid, Copolymerisate aus Ethylenoxid und Butylenoxid sowie Copolymerisate aus Ethylenoxid, Propylenoxid und mindestens einem Butylenoxid. Als Komponente d1) eignen sich auch die Allylether der zuvor genannten Polyalkylenglykole.

Verzweigte polyetherhaltige Polymerisate d1) können hergestellt werden, indem man beispielsweise an Polyalkoholreste, z. B. an Pentaerythrit, Glycerin oder an Zuckeralkohole wie D-Sorbit und D-Mannit oder an Polysaccharide wie Cellulose und Stärke, wenigstens eines der zuvor genannten Alkylenoxide anlagert. Die Alkylenoxid-Einheiten können im Anlagerungsprodukt statistisch verteilt sein oder in Form von Blöcken vorliegen.

Es ist auch möglich, Polyester von Polyalkylenoxiden und aliphatischen oder aromatischen Dicarbonsäuren, z. B. Oxalsäure, Bernsteinsäure, Adipinsäure und Terephthalsäure als polyetherhaltige Verbindung d1) zu verwenden. Geeignete Polyester von Polyalkylenoxiden mit Molmassen von 1500 bis 25000 sind z. B. beschrieben in EP-A-0 743 962. Des Weiteren können auch Polycarbonate aus der Umsetzung von Polyalkylenoxiden mit Phosgen oder mit Carbonaten, wie z. B. Diphenylcarbonat, sowie Polyurethane aus der Umsetzung von Polyalkylenoxiden mit aliphatischen und aromatischen Diisocyanaten als Verbindung d1) verwendet werden.

Nach einer bevorzugten Ausführungsform wird zur Herstellung der ampholytischen Copolymere eine Komponente d1) eingesetzt, die wenigstens ein Polyetherurethan umfasst.

Geeignete Polyetherurethane sind die Kondensationsprodukte von Polyetherpolyolen, wie Polyetherdiolen, mit Polyisocyanaten, wie Diisocyanaten. Geeignete Polyetherpolyole sind die zuvor genannten Polyalkylenglykole, die beispielsweise aus der Polymerisation von cyclischen Ethern, wie Tetrahydrofuran, oder aus der Umsetzung von einem oder mehreren Alkylenoxiden mit einem Startermolekül, das zwei oder mehr aktive Wasserstoffatome aufweist, erhältlich sind.

Geeignete Polyisocyanate sind ausgewählt unter Verbindungen mit 2 bis 5 Isocyanatgruppen, Isocyanatpräpolymeren mit einer mittleren Anzahl von 2 bis 5 Isocyanatgruppen, und Mischungen davon. Dazu zählen z. B. aliphatische, cycloaliphatische und aromatische Di-, Tri- und Polyisocyanate. Geeignete Diisocyanate sind z. B. Tetramethylendiisocyanat, Hexamethylendiisocyanat, 2,3,3-Trimethylhexamethylendiisocyanat, 1,4-Cyclohexylendiisocyanat, Isophorondiisocyanat, 1,4-Phenylendiisocyanat, 2,4- und 2,6-Toluylendiisocyanat und deren Isomerengemische (z. B. 80 % 2,4- und 20 % 2,6-Isomer), 1,5-Naphthylendiisocyanat, 2,4- und 4,4'-Diphenylmethandiisocyanat. Ein geeignetes Triisocyanat ist z. B. Triphenylmethan-4,4',4"-triisocyanat. Weiterhin geeignet sind Isocyanatpräpolymere und Polyisocyanate, die durch Addition der zuvor genannten Isocyanate an polyfunktionelle hydroxyl- oder amingruppenhaltige Verbindungen erhältlich sind. Weiterhin geeignet sind Polyisocyanate, die durch Biuret- oder Isocyanuratbildung entstehen. Bevorzugt werden Hexamethylendiisocyanat, trimerisiertes Hexamethylendiisocyanat, Isophorondiisocyanat, 2,4-Toluylendiisocyanat, 2,6-Toluylendiisocyanat, und Mischungen davon, eingesetzt.

Nach einer weiteren bevorzugten Ausführungsform wird zur Herstellung der ampholytischen Copolymere eine Komponente d1) eingesetzt, die wenigstens ein Polyalkylenoxid-haltiges Silikonderivat umfasst.

Geeignete Silikonderivate d1) sind die unter den INCI-Namen Dimethicone-Copolyole oder Silikontenside bekannten Verbindungen, wie zum Beispiel die unter den Markennamen Abil^{®} (der Fa. T. Goldschmidt), Alkasil^{®} (der Fa. Rhône-Poulenc), Silicone Polyol Copolymer^{®} (der Fa Genesee), Belsil^{®} (der Fa. Wacker), Silwet^{®} (der Fa. OSI) oder Dow Corning (der Fa. Dow Corning) erhältlich Verbindungen. Diese beinhalten Verbindungen mit den CAS-Nummern 64365-23-7; 68937-54-2; 68938-54-5; 68937-55-3.

Besonders geeignete Verbindungen d1) sind solche, die die folgenden Strukturelemente enthalten: 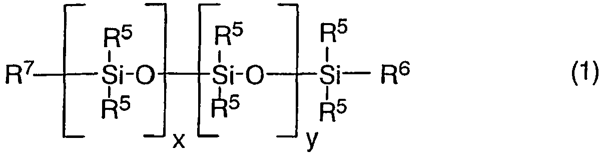 wobei: 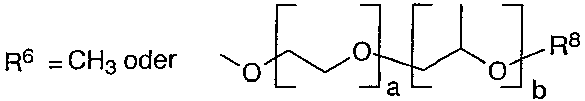 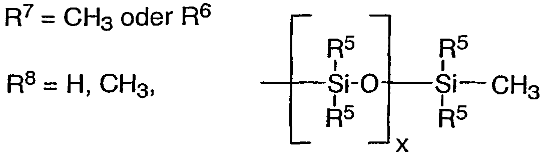 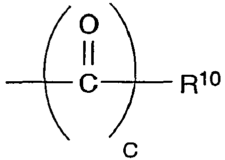
R¹⁰ ein organischer Rest aus 1 bis 40 Kohlenstoffatomen, der Amino-, Carbonsäure- oder Sulfonatgruppen enthalten kann oder für den Fall c = O, auch das Anion einer anorganischen Säure bedeutet,
   und wobei die Reste R⁵ identisch oder unterschiedlich sein können, und entweder aus der Gruppe der aliphatischen Kohlenwasserstoffe mit 1 bis 20 Kohlenstoffatomen stammen, cyclische aliphatische Kohlenwasserstoffe mit 3 bis 20 C-Atomen sind, aromatischer Natur oder gleich R⁹ sind, wobei: 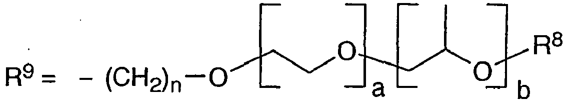 und n eine ganze Zahl von 1 bis 6 ist,
x und y ganze Zahlen derart sind, dass das Molekulargewicht des Polysiloxan-Blocks zwischen 300 und 30000 liegt,
a,b ganze Zahlen zwischen 0 und 50 sein können mit der Maßgabe, dass die Summe aus a und b größer als 0 ist, und c 0 oder 1 ist.

Bevorzugte Reste R⁶ und R⁹ sind solche, bei denen die Summe aus a+b zwischen 5 und 30 beträgt.

Bevorzugt werden die Gruppen R⁵ aus der folgenden Gruppe ausgewählt: Methyl, Ethyl,Propyl, Butyl, Isobutyl, Pentyl, Isopentyl, Hexyl, Octyl, Decyl, Dodecyl und Octadecyl, cycloaliphatische Reste, speziell Cyclohexyl, aromatische Gruppen, speziell Phenyl oder Naphthyl, gemischt aromatisch-aliphatische Reste wie Benzyl oder Phenylethyl sowie Tolyl und Xylyl und _{R}⁹.

Besonders geeignete Reste R⁸ sind solche, bei denen im Falle von R⁸ = -(CO)_{c}-R¹⁰ R¹⁰ ein beliebiger Alkyl-, Cycloalkyl oder Arylrest bedeutet, der zwischen 1 und 40 C-Atomen besitzt und der weitere ionogene Gruppen wie NH₂, COOH, SO₃H tragen kann.

Bevorzugte anorganische Reste R¹⁰ sind, für den Fall c = O, Phosphat und Sulfat.

Besonders bevorzugte Silikonderivate d) sind solche, der allgemeinen Struktur:

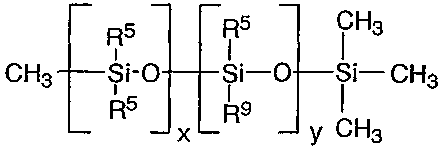

Als Propfgrundlage eignen sich vorzugsweise weiterhin Polymerisate d2), die mindestens 50 Gew.-% an Vinylalkoholeinheiten besitzen. Bevorzugt enthalten diese Polymerisate mindestens 70 Gew.-%, ganz besonders bevorzugt 80 Gew.-% Polyvinylalkoholeinheiten. Solche Polymerisate werden üblicherweise durch Polymerisation eines Vinylesters und anschließender zumindest teilweiser Alkoholyse, Aminolyse oder Hydrolyse hergestellt. Bevorzugt sind Vinylester linearer und verzweigter C₁-C₁₂-Carbonsäuren, ganz besonders bevorzugt ist Vinylacetat. Die Vinylester können selbstverständlich auch im Gemisch eingesetzt werden.

Als Comonomere des Vinylesters zur Synthese der Pfropfgrundlage d2) kommen beispielsweise N-Vinylcaprolactam, N-Vinylpyrrolidon, N-Vinylimidazol, N-Vinyl-2-methylimidazol, N-Vinyl-4-methylimidazol, 3-Methyl-1-vinylimidazoliumchlorid, 3-Methyl-1-vinylimidazoliummethylsulfat, Diallylammoniumchlorid, Styrol, Alkylstyrole in Frage.

Weitere geeignete Comonomere zur Herstellung der Pfropfgrundlage d2) sind beispielsweise monoethylenisch ungesättigte C₃-C₆-Carbonsäuren wie z. B. Acrylsäure, Methacrylsäure, Crotonsäure, Fumarsäure, sowie deren Ester, Amide und Nitrile wie z. B. Acrylsäuremethylester, Acrylsäureethylester, Methacrylsäuremethylester, Methacrylsäureethylester, Methacrylsäurestearylester, Hydroxyethylacrylat, Hydroxypropylacrylat, Hydroxybutylacrylat, Hydroxyethylmethacrylat, Hydroxypropylmethacrylat, Hydroxyisobutylacrylat, Hydroxyisobutylmethacrylat, Maleinsäuremonomethylester, Maleinsäuredimethylester, Maleinsäuremonoethylester, Maleinsäurediethylester, 2-Ethylhexylacrylat, 2-Ethylhexylmethacrylat, Maleinsäureanhydrid sowie dessen Halbester, Alkylenglykol(meth)acrylate, Acrylamid, Methacrylamid, N-Dimethylacrylamid, N-tert.-butylacrylamid, Acrylnitril, Methacrylnitril, Vinylether wie z. B. Methyl-, Ethyl-, Butyl oder Dodecylvinylether, kationische Monomere wie Dialkylaminoalkyl(meth)acrylate und Dialkylaminoalkyl(meth)acrylamide wie Dimethylaminoethylacrylat, Diethylaminoethylacrylat, Diethylaminoethylmethacrylat, sowie die Salze der zuletzt genannten Monomeren mit Carbonsäuren oder Mineralsäuren sowie die quarternierten Produkte.

Bevorzugte Propfgrundlagen d2) sind Polymerisate, die durch Homopolymerisation von Vinylacetat und anschließender zumindest teilweiser Hydrolyse, Alkoholyse oder Aminolyse hergestellt werden.

Besonders bevorzugte Propfgrundlagen d2) sind Polymerisate, die durch Homopolymerisation von Vinylacetat und anschließender zumindest teilweiser Verseifung hergestellt werden. Solche Polyvinylalkoholeinheiten enthaltenden Polymere sind unter dem Namen Mowiol® erhältlich.

Bevorzugt werden als Komponente d) Stärke und/oder Stärkederivate d3) eingesetzt. Dazu zählen Substanzen, die Saccharid-Strukturen enthalten. Solche natürlichen Substanzen sind beispielsweise Saccharide pflanzlicher oder tierischer Herkunft oder Produkte, die durch Metabolisierung durch Mikroorganismen entstanden sind, sowie deren Abbauprodukte. Geeignete Pfropfgrundlagen d3) sind beispielsweise Oligosaccharide, Polysaccharide, oxidativ, enzymatisch oder hydrolytisch abgebaute Polysaccharide, oxidativ hydrolytisch abgebaute oder oxidativ enzymatisch abgebaute Polysaccharide, chemisch modifizierte Oligo- oder Polysaccharide und Mischungen davon. Bevorzugte Produkte sind die in US 5,334,287 in Spalte 4, Zeile 20 bis Spalte 5, Zeile 45 genannten Verbindungen.

Geeignete kommerziell erhältliche Produkte sind die C-Pur^{®} und C-Dry^{®}-Marken der Fa. Cerestar.

Gewünschtenfalls können Gemische von Verbindungen der Komponente d) eingesetzt werden.

Eine bevorzugte Ausführungsform sind Copolymere, die durch Copolymerisation in Gegenwart wenigstens einer Verbindung d1) erhältlich sind, die ausgewählt ist unter Polyalkylenoxiden, polyalkylenoxidhaltigen Silikonderivaten und Mischungen davon.

Vorzugsweise weisen die erfindungsgemäßen Copolymere einen K-Wert (gemessen nach E. Fikentscher, Cellulose-Chemie 13 (1932), S. 58-64) an einer 1 gew.-%igen Lösung in Wasser im Bereich von etwa 30 bis 300, besonders bevorzugt 40 bis 150 auf.

Je nach K-Wert eignen sich die erfindungsgemäßen Polymere für eine Vielzahl kosmetischer und pharmazeutischer Anwendungen. So lassen sich Polymere mit einem K-Wert bis etwa 50 vorteilhaft als Sprays (Aerosol- und Pumpsprays) formulieren. Polymere mit einem K-Wert in einem Bereich von etwa 50 bis 90 eignen sich vorteilhaft für Gele und Schäume. Für Shampoos und hautkosmetische Anwendungen eignen sich bevorzugt Polymere mit einem K-Wert von mindestens 80.

Bevorzugte Polymere sind erhältlich durch radikalische Polymerisation von
- 1 bis 25 Gew.-%, bezogen auf das Gesamtgewicht der zur Polymerisation eingesetzten Komponenten, wenigstens eines Polyallylpolyetherurethans,
- 50 bis 99 Gew.-% wenigstens einer radikalisch polymerisierbaren nichtionischen Verbindung M1),
- 0 bis 25 Gew.-% wenigstens eines Monomers M2) mit mindestens einer ionogenen und/oder ionischen Gruppe pro Molekül,
- 0 bis 10 Gew.-% wenigstens eines Vernetzers,
gegebenenfalls in Gegenwart von bis zu 25 Gew.-% wenigstens einer Komponente d), wie zuvor definiert.

Die Herstellung der Copolymere A) erfolgt nach üblichen, dem Fachmann bekannten Verfahren, vorzugsweise durch Lösungs-Polymerisation und Fällungs-Polymerisation.

Bevorzugte Lösemittel zur Lösungspolymerisation sind wässrige Lösungsmittel, wie Wasser und Gemische aus Wasser mit wassermischbaren Lösungsmitteln, beispielsweise Alkoholen, wie Methanol, Ethanol, n-Propanol, Isopropanol, n-Butanol, sek.-Butanol, tert.-Butanol, n-Hexanol und Cyclohexanol sowie Glykole, wie Ethylenglykol, Propylenglykol und Butylenglykol sowie die Methyl- oder Ethylether der zweiwertigen Alkohole, Diethylenglykol, Triethylenglykol, Glycerin und Dioxan. Besonders bevorzugt ist die Polymerisation in Wasser oder einem Wasser/Alkohol-Gemisch, beispielsweise in einem Wasser/Ethanol-Gemisch. Das Verhältnis von Alkohol zu Wasser liegt in solchen Gemischen bevorzugt in einem Bereich von 1:1 bis 1:7 Vol.-%.

Die Fällungspolymerisation erfolgt beispielsweise in einem Ester, wie Essigsäureethylester oder Essigsäurebutylester als Lösungsmittel. Die resultierenden Polymerteilchen fallen aus der Reaktionslösung aus und können durch übliche Verfahren, wie Filtration mittels Unterdruck, isoliert werden. Bei der Fällungspolymerisation werden in der Regel Polymere mit höheren Molekulargewichten als bei der Lösungspolymerisation erhalten.

Die Polymerisationstemperaturen liegen vorzugsweise in einem Bereich von etwa 30 bis 120 °C, besonders bevorzugt 40 bis 100 °C. Die Polymerisation erfolgt üblicherweise unter atmosphärischem Druck, sie kann jedoch auch unter vermindertem oder erhöhtem Druck ablaufen. Ein geeigneter Druckbereich liegt zwischen 1 und 5 bar.

Zur Herstellung der Polymerisate können das Polyallylpolyetherurethan und die Monomere gegebenenfalls in Gegenwart der Komponente d) mit Hilfe von Radikale bildenden Initiatoren polymerisiert werden.

Als Initiatoren für die radikalische Polymerisation können die hierfür üblichen Peroxo- und/oder Azo-Verbindungen eingesetzt werden, beispielsweise Alkali- oder Ammoniumperoxidisulfate, Diacetylperoxid, Dibenzoylperoxid, Succinylperoxid, Di-tert.-butylperoxid, tert.-Butylperbenzoat, tert.-Butylperpivalat, tert.-Butylperoxy-2-ethylhexanoat, tert.-Butylpermaleinat, Cumolhydroperoxid, Diisopropylperoxidicarbamat, Bis-(o-toluoyl)-peroxid, Didecanoylperoxid, Dioctanoylperoxid, Dilauroylperoxid, tert.-Butylperisobutyrat, tert.-Butylperacetat, Di-tert.-Amylperoxid, tert.-Butylhydroperoxid, Azo-bis-isobutyronitril, Azo-bis-(2-amidinopropan)dihydrochlorid oder 2-2'-Azo-bis-(2-methyl-butyronitril). Geeignet sind auch Initiatormischungen oder Redox-Initiator-Systeme, wie z. B. Ascorbinsäure/Eisen(II)sulfat/Natriumperoxodisulfat, tert.-Butylhydroperoxid/Natriumdisulfit, tert.-Butylhydroperoxid/Natriumhydroxymethansulfinat, H₂O₂/Cu^{I}.

Die verwendeten Mengen an Initiator bzw. Initiatorgemischen bezogen auf eingesetzte Monomere liegen im Allgemeinen zwischen 0,01 und 10 Gew.-%, vorzugsweise zwischen 0,1 und 5 Gew.-%.

Zur Einstellung des Molekulargewichts kann die Polymerisation in Gegenwart wenigstens eines Reglers erfolgen. Als Regler können die üblichen, dem Fachmann bekannten Verbindungen, wie z. B. Schwefelverbindungen, z. B. Mercaptoethanol, 2-Ethylhexylthioglycolat, Thioglycolsäure oder Dodecylmercaptan sowie Tribromchlormethan oder andere Verbindungen, die regelnd auf das Molekulargewicht der erhaltenen Polymerisate wirken, eingesetzt werden. Ein bevorzugter Regler ist Cystein.

Zur Erzielung möglichst reiner Polymere mit geringem Restmonomergehalt kann sich an die Polymerisation (Hauptpolymerisation) ein Nachpolymerisationsschritt anschließen. Die Nachpolymerisation kann in Gegenwart desselben oder eines anderen Initiatorsystems wie die Hauptpolymerisation erfolgen. Vorzugsweise erfolgt die Nachpolymerisation mindestens bei der gleichen, vorzugsweise bei einer höheren Temperatur als die Hauptpolymerisation. Die Temperatur bei der Haupt- und der Nachpolymerisation beträgt vorzugsweise höchstens 90 °C. Bevorzugt wird der Reaktionsansatz zwischen dem ersten und dem zweiten Polymerisationsschritt einem Strippen mit Wasserdampf oder einer Wasserdampf-Destillation unterzogen.

Wird bei der Herstellung der Polymere ein organisches Lösungsmittel eingesetzt, so kann dieses durch übliche, dem Fachmann bekannte Verfahren, z. B. durch Destillation bei vermindertem Druck, entfernt werden.

Die Polymerisation erfolgt vorzugsweise bei einem pH-Wert im Bereich von 5,5 bis 8,0, besonders bevorzugt von 5,6 bis 7,5 und insbesondere von 5,8 bis 7,3. Dies führt in der Regel zur Erzielung möglichst reiner Polymere mit geringem Restmonomergehalt, was eventuell darauf zurückzuführen ist, dass Amine, die als Abspaltprodukt gebildet werden und die unter den Polymerisationsbedingungen gegebenenfalls mit einigen Monomeren zu unerwünschten Nebenprodukten reagieren können, entfernt werden. Die Einstellung des pH-Wertes erfolgt durch Zugabe einer geeigneten Säure, wie Milchsäure, Weinsäure, Phosphorsäure oder durch Zugabe einer Base, vorzugsweise eines Amins, und insbesondere eines Aminoalkohols, wie Triethanolamin, Methyldiethanolamin, Dimethylethanolamin oder 2-Amino-2-methylpropanol.

Produkte mit besonders hoher Reinheit und entsprechend vorteilhaften Eigenschaften für einen Einsatz in der Kosmetik können erzielt werden, wenn das Reaktionsprodukt nach der Polymerisation, gegebenenfalls vor und/oder nach einer Nachpolymerisation, einer Wasserdampfdestillation bzw. einem Strippen mit Wasserdampf unterzogen wird. Auch diese Behandlung mit Wasserdampf dient z. B. der Entfernung von Aminen und weiterer unerwünschter, mit Wasserdampf entfernbarer Nebenprodukte aus dem Reaktionsgemisch. Vorzugsweise erfolgt die Wasserdampf-Behandlung zumindest zwischen Haupt- und Nachpolymerisation. Der pH-Wert des Polymerisationsprodukts wird vorzugsweise vor der Wasserdampf-Behandlung auf einen Wert von höchstens 6,5 eingestellt. Die Temperatur des eingesetzten Wasserdampfs und der behandelten Polymerlösung beträgt vorzugsweise mindestens 90 °C.

Die Polymerlösungen können durch verschiedene Trocknungsverfahren, wie z. B. Sprühtrocknung, Fluidized Spray Drying, Walzentrocknung oder Gefriertrocknung in Pulverform überführt werden. Bevorzugt wird die Sprühtrocknung eingesetzt. Die so erhaltenen Polymer-Trockenpulver lassen sich vorteilhafterweise durch Lösen bzw. Redispergieren in Wasser erneut in eine wässrige Lösung bzw. Dispersion überführen. Pulverförmige Copolymere haben den Vorteil einer besseren Lagerfähigkeit, einer einfacheren Transportmöglichkeit und zeigen in der Regel eine geringere Neigung für Keimbefall.

Ein weiterer Gegenstand der Erfindung ist ein kosmetisches oder pharmazeutisches Mittel, enthaltend
A) wenigstens ein wasserlösliches oder wasserdispergierbares Polymer, wie zuvor definiert, und
B) wenigstens einen kosmetisch akzeptablen Träger.

Der kosmetisch akzeptable Träger B) ist vorzugsweise ausgewählt unter
i) Wasser,
ii) wassermischbaren organischen Lösungsmitteln, vorzugsweise C₁-C₄-Alkanolen,
iii)Ölen, Fetten, Wachsen,
iv) von iii) verschiedenen Estern von C₆-C₃₀-Monocarbonsäuren mit ein-, zwei- oder dreiwertigen Alkoholen,
v) gesättigten acyclischen und cyclischen Kohlenwasserstoffen,
vi) Fettsäuren,
vii)Fettalkoholen
und Mischungen davon.

Die erfindungsgemäßen Mittel weisen z. B. eine Öl- bzw. Fettkomponente B) auf, die ausgewählt ist unter: Kohlenwasserstoffen geringer Polarität, wie Mineralölen; linearen gesättigten Kohlenwasserstoffen, vorzugsweise mit mehr als 8 C-Atomen, wie Tetradecan, Hexadecan, Octadecan etc.; cyclischen Kohlenwasserstoffen, wie Decahydronaphthalin; verzweigten Kohlenwasserstoffen; tierischen und pflanzlichen Ölen; Wachsen; Wachsestern; Vaselin; Estern, bevorzugt Estern von Fettsäuren, wie z. B. die Ester von C₁-C₂₄-Monoalkoholen mit C₁-C₂₂-Monocarbonsäuren, wie Isopropylisostearat, n-Propylmyristat, iso-Propylmyristat, n-Propylpalmitat, iso-Propylpalmitat, Hexacosanylpalmitat, Octacosanylpalmitat, Triacontanylpalmitat, Dotriacontanylpalmitat, Tetratriacontanylpalmitat, Hexacosanylstearat, Octacosanylstearat, Triacontanylstearat, Dotriacontanylstearat, Tetratriacontanylstearat; Salicylaten; wie C₁-C₁₀-Salicylaten, z. B. Octylsalicylat; Benzoatestern, wie C₁₀-C₁₅-Alkylbenzoaten, Benzylbenzoat; anderen kosmetischen Estern, wie Fettsäuretriglyceriden, Propylenglykolmonolaurat, Polyethylenglykolmonolaurat, C₁₀-C₁₅-Alkyllactaten, etc. und Mischungen davon.

Geeignete Siliconöle B) sind z. B. lineare Polydimethylsiloxane, Poly(methylphenylsiloxane), cyclische Siloxane und Mischungen davon. Das zahlenmittlere Molekulargewicht der Polydimethylsiloxane und Poly(methylphenylsiloxane) liegt vorzugsweise in einem Bereich von etwa 1000 bis 150000 g/mol. Bevorzugte cyclische Siloxane weisen 4- bis 8-gliedrige Ringe auf. Geeignete cyclische Siloxane sind z. B. unter der Bezeichnung Cyclomethicon kommerziell erhältlich.

Bevorzugte Öl- bzw. Fettkomponenten B) sind ausgewählt unter Paraffin und Paraffinölen; Vaselin; natürlichen Fetten und Ölen, wie Castoröl, Sojaöl, Erdnussöl, Olivenöl, Sonnenblumenöl, Sesamöl, Avocadoöl, Kakaobutter, Mandelöl, Pfirsichkernöl, Ricinusöl, Lebertran, Schweineschmalz, Walrat, Spermacetöl, Spermöl, Weizenkeimöl, Macadamianussöl, Nachtkerzenöl, Jojobaöl; Fettalkoholen, wie Laurylalkohol, Myristylalkohol, Cetylalkohol, Stearylalkohol, Oleylalkohol, Cetylalkohol; Fettsäuren, wie Myristinsäure, Stearinsäure, Palmitinsäure, Ölsäure, Linolsäure, Linolensäure und davon verschiedenen gesättigten, ungesättigten und substituierten Fettsäuren; Wachsen, wie Bienenwachs, Carnaubawachs, Candilillawachs, Walrat sowie Mischungen der zuvor genannten öl- bzw. Fettkomponenten.

Geeignete kosmetisch und pharmazeutisch verträgliche Öl- bzw. Fettkomponenten B) sind in Karl-Heinz Schrader, Grundlagen und Rezepturen der Kosmetika, 2. Auflage, Verlag Hüthig, Heidelberg, S. 319-355 beschrieben, worauf hier Bezug genommen wird.

Geeignete hydrophile Träger B) sind ausgewählt unter Wasser, 1-, 2- oder mehrwertigen Alkoholen mit vorzugsweise 1 bis 8 Kohlenstoffatomen, wie Ethanol, n-Propanol, iso-Propanol, Propylenglycol, Glycerin, Sorbit, etc.

Bei den erfindungsgemäßen kosmetischen Mitteln kann es sich um hautkosmetische, dermatologische oder haarkosmetische Mittel handeln.

Vorzugsweise liegen die erfindungsgemäßen Mittel in Form einer (niedrig- bis hochviskosen) Lösung eines Gels, Wachses, Schaums, Sprays, einer Salbe, Creme, Emulsion, Suspension, Lotion, Milch oder Paste vor. Gewünschtenfalls können auch Liposomen oder Mikrosphären eingesetzt werden.

Die erfindungsgemäßen kosmetisch oder pharmazeutisch aktiven Mittel können zusätzlich kosmetisch und/oder dermatologisch aktive Wirkstoffe sowie Hilfsstoffe enthalten.

Vorzugsweise enthalten die erfindungsgemäßen kosmetischen Mittel wenigstens ein wie vorstehend definiertes Copolymer A, wenigstens einen wie vorstehend definierten Träger B und wenigstens einen, von Copolymer A verschiedenen Bestandteil, der ausgewählt ist unter kosmetisch aktiven Wirkstoffen, Emulgatoren, Tensiden, Konservierungsmitteln, Parfümölen, Verdickern, Haarpolymeren, Haar - und Hautconditionern, Pfropfpolymeren, wasserlöslichen oder dispergierbaren silikonhaltigen Polymeren, Lichtschutzmitteln, Bleichmitteln, Gelbildnern, Pflegemitteln, Färbemitteln, Tönungsmitteln, Bräunungsmitteln, Farbstoffen, Pigmenten, Konsistenzgebern, Feuchthaltemitteln, Rückfettern, Collagen, Eiweißhydrolysaten, Lipiden, Antioxidantien, Entschäumern, Antistatika, Emollienzien, Weichmachern.

Geeignete kosmetisch und/oder dermatologisch aktive Wirkstoffe sind z. B. färbende Wirkstoffe, Haut- und Haarpigmentierungsmittel, Tönungsmittel, Bräunungsmittel, Bleichmittel, Keratin-härtende Stoffe, antimikrobielle Wirkstoffe, Lichtfilterwirkstoffe, Repellentwirkstoffe, hyperemisierend wirkende Stoffe, keratolytisch und keratoplastisch wirkende Stoffe, Antischuppenwirkstoffe, Antiphlogistika, keratinisierend wirkende Stoffe, antioxidativ bzw. als Radikalfänger aktive Wirkstoffe, hautbefeuchtende oder -feuchthaltende Stoffe, rückfettende Wirkstoffe, antierythimatös oder antiallergisch aktive Wirkstoffe und Mischungen davon.

Künstlich hautbräunende Wirkstoffe, die geeignet sind, die Haut ohne natürliche oder künstliche Bestrahlung mit UV-Strahlen zu bräunen, sind z. B. Dihydroxyaceton, Alloxan und Walnussschalenextrakt. Geeignete Keratin-härtende Stoffe sind in der Regel Wirkstoffe, wie sie auch in Antitranspirantien eingesetzt werden, wie z. B. Kaliumaluminiumsulfat, Aluminiumhydroxychlorid, Aluminiumlactat, etc. Antimikrobielle Wirkstoffe werden eingesetzt, um Mikroorganismen zu zerstören bzw. ihr Wachstum zu hemmen und dienen somit sowohl als Konservierungsmittel als auch als desodorierend wirkender Stoff, welcher die Entstehung oder die Intensität von Körpergeruch vermindert. Dazu zählen z. B. übliche, dem Fachmann bekannte Konservierungsmittel, wie p-Hydroxybenzoesäureester, Imidazolidinyl-Harnstoff, Formaldehyd, Sorbinsäure, Benzoesäure, Salicylsäure, etc. Derartige desodorierend wirkende Stoffe sind z. B. Zinkricinoleat, Triclosan, Undecylensäurealkylolamide, Citronensäuretriethylester, Chlorhexidin etc. Geeignete Lichtfilterwirkstoffe sind Stoffe, die UV-Strahlen im UV-B- und/oder UV-A-Bereich absorbieren. Geeignete UV-Filter sind z. B. 2,4,6-Triaryl-1,3,5-triazine, bei denen die Arylgruppen jeweils wenigstens einen Substituenten tragen können, der vorzugsweise ausgewählt ist unter Hydroxy, Alkoxy, speziell Methoxy, Alkoxycarbonyl, speziell Methoxycarbonyl und Ethoxycarbonyl und Mischungen davon. Geeignet sind weiterhin p-Aminobenzoesäureester, Zimtsäureester, Benzophenone, Campherderivate sowie UV-Strahlen abhaltende Pigmente, wie Titandioxid, Talkum und Zinkoxid. Geeignete Repellentwirkstoffe sind Verbindungen, die in der Lage sind, bestimmte Tiere, insbesondere Insekten, vom Menschen abzuhalten oder zu vertreiben. Dazu gehört z. B. 2-Ethyl-1,3-hexandiol, N,N-Diethyl-m-toluamid etc. Geeignete hyperemisierend wirkende Stoffe, welche die Durchblutung der Haut anregen, sind z. B. ätherische Öle, wie Latschenkiefer, Lavendel, Rosmarin, Wacholderbeer, Rosskastanienextrakt, Birkenblätterextrakt, Heublumenextrakt, Ethylacetat, Campher, Menthol, Pfefferminzöl, Rosmarinextrakt, Eukalyptusöl, etc. Geeignete keratolytisch und keratoplastisch wirkende Stoffe sind z. B. Salicylsäure, Kalziumthioglykolat, Thioglykolsäure und ihre Salze, Schwefel, etc. Geeignete Antischuppen-Wirkstoffe sind z. B. Schwefel, Schwefelpolyethylenglykolsorbitanmonooleat, Schwefelricinolpolyethoxylat, Zinkpyrithion, Aluminiumpyrithion, etc. Geeignete Antiphlogistika, die Hautreizungen entgegenwirken, sind z. B. Allantoin, Bisabolol, Dragosantol, Kamillenextrakt, Panthenol, etc.

Die erfindungsgemäßen kosmetischen Mittel können als kosmetischen und/oder pharmazeutischen Wirkstoff (wie auch gegebenenfalls als Hilfsstoff) wenigstens ein kosmetisch oder pharmazeutisch akzeptables von Verbindungen der Komponente A) verschiedenes Polymer enthalten. Dazu zählen ganz allgemein anionische, kationische, amphotere und neutrale Polymere. Diese sind vorzugsweise wasserlöslich oder zumindest wasserdispergierbar.

Beispiele für anionische Polymere sind Homo- und Copolymerisate von Acrylsäure und Methacrylsäure oder deren Salze, Copolymere von Acrylsäure und Acrylamid und deren Salze; Natriumsalze von Polyhydroxycarbonsäuren, wasserlösliche oder wasserdispergierbare Polyester, Polyurethane, z. B. Luviset PUR® der Fa. BASF, und Polyharnstoffe. Besonders geeignete Polymere sind Copolymere aus t-Butylacrylat, Ethylacrylat, Methacrylsäure (z. B. Luvimer® 100P), Copolymere aus Ethylacrylat und Methacrylsäure (z. B. Luviumer^{®} MAE), Copolymere aus N-tert.-Butylacrylamid, Ethylacrylat, Acrylsäure (Ultrahold^{®} 8, strong), Copolymere aus Vinylacetat, Crotonsäure und gegebenenfalls weitere Vinylester (z. B. Luviset^{®} Marken), Maleinsäureanhydridcopolymere, gegebenenfalls mit Alkohol umgesetzt, anionische Polysiloxane, z. B. carboxyfunktionelle, t-Butylacrylat, Methacrylsäure (z. B. Luviskol^{®} VBM), Copolymere von Acrylsäure und Methacrylsäure mit hydrophoben Monomeren, wie z. B. C₄-C₃₀-Alkylester der Meth(acrylsäure), C₄-C₃₀-Alkylvinyle-ster, C₄-C₃₀-Alkylvinylether und Hyaluronsäure. Beispiele für anionische Polymere sind weiterhin Vinylacetat/Crotonsäure-Copolymere, wie sie beispielsweise unter den Bezeichnungen Resyn® (National Starch) und Gafset^{®} (GAF) im Handel sind und Vinylpyrrolidon/Vinylacrylat-Copolymere, erhältlich beispielsweise unter dem Warenzeichen Luviflex^{®} (BASF). Weitere geeignete Polymere sind das unter der Bezeichnung Luviflex^{®} VBM-35 (BASF) erhältliche Vinylpyrrolidon/Acrylat-Terpolymer und natriumsulfonathaltige Polyamide oder natriumsulfonathaltige Polyester.

Weitere geeignete Polymere sind kationische Polymere mit der Bezeichnung Polyquaternium nach INCI, z. B. Copolymere aus Vinylpyrrolidon/N-Vinylimidazoliumsalzen (Luviquat^{®} FC, Luviquat^{®} HM, Luviquat^{®} MS, Luviquat^{®} Care), Copolymere aus N-Vinylpyrrolidon/Dimethylaminoethylmethacrylat, quaternisiert mit Diethylsulfat (Luviquat^{®} PQ 11), Copolymere aus N-Vinylcaprolactam/N-Vinylpyrrolidon/N-Vinylimidazoliumsalzen (Luviquat^{®} Hold); kationische Cellulosederivate (Polyquaternium-4 und -10), Acrylamidocopolymere (Polyquaternium-7) und Chitosan. Geeignete kationische (quaternisierte) Polymere sind auch Merquat^{®} (Polymer auf Basis von Dimethyldiallylammoniumchlorid), Gafquat^{®} (quaternäre Polymere, die durch Reaktion von Polyvinylpyrrolidon mit quaternären Ammoniumverbindungen entstehen), Polymer JR (Hydroxyethylcellulose mit kationischen Gruppen) und kationische Polymere auf pflanzlicher Basis, z. B. Guarpolymere, wie die Jaguar^{®}-Marken der Fa. Rhodia. Geeignet sind auch die in der deutschen Patentanmeldung P 102 43 573.1 beschriebenen Polymere mit (Meth)acrylsäureamideinheiten.

Weitere geeignete Polymere sind auch neutrale Polymere, wie Polyvinylpyrrolidone, Copolymere aus N-Vinylpyrrolidon und Vinylacetat und/oder Vinylpropionat, Polysiloxane, Polyvinylcaprolactam und andere Copolymere mit N-Vinylpyrrolidon, Polyethylenimine und deren Salze, Polyvinylamine und deren Salze, Cellulosederivate, Polyasparaginsäuresalze und Derivate. Dazu zählt beispielsweise Luviflex^{®} Swing (teilverseiftes Copolymerisat von Polyvinylacetat und Polyethylenglykol, Fa. BASF).

Geeignete Polymere sind auch nichtionische, wasserlösliche bzw. wasserdispergierbare Polymere oder Oligomere, wie Polyvinylcaprolactam, z. B. Luviskol^{®} Plus (BASF), oder Polyvinylpyrrolidon und deren Copolymere, insbesondere mit Vinylestern, wie Vinylacetat, z. B. Luviskol^{®} VA 37 (BASF); Polyamide, z. B. auf Basis von Itaconsäure und aliphatischen Diaminen, wie sie z. B. in der DE-A-43 33 238 beschrieben sind.

Geeignete Polymere sind auch amphotere oder zwitterionische Polymere, wie die unter den Bezeichnungen Amphomer^{®} (National Starch) erhältlichen Octylacrylamid/Methylmethacrylat/tert.-Butylaminoethylmethacrylat/2-Hydroxypropylmethacrylat-Copolymere sowie zwitterionische Polymere, wie sie beispielsweise in den deutschen Patentanmeldungen DE 39 29 973, DE 21 50 557, DE 28 17 369 und DE 37 08 451 offenbart sind. Acrylamidopropyltrimethylammoniumchlorid/Acrylsäure- bzw. -Methacrylsäure-Copolymerisate und deren Alkali- und Ammoniumsalze sind bevorzugte zwitterionische Polymere. Weiterhin geeignete zwitterionische Polymere sind Methacroylethylbetain/Methacrylat-Copolymere, die unter der Bezeichnung Amersette® (AMERCHOL) im Handel erhältlich sind, und Copolymere aus Hydroxyethylmethacrylat, Methylmethacrylat, N,N-Dimethylaminoethylmethacrylat und Acrylsäure (Jordapon^{®}).

Geeignete Polymere sind auch nichtionische, siloxanhaltige, wasserlösliche oder -dispergierbare Polymere, z. B. Polyethersiloxane, wie Tegopren^{®} (Fa. Goldschmidt) oder Belsil^{®} (Fa. Wacker).

Die Formulierungsgrundlage erfindungsgemäßer pharmazeutischer Mittel enthält bevorzugt pharmazeutisch akzeptable Hilfsstoffe. Pharmazeutisch akzeptabel sind die im Bereich der Pharmazie, der Lebensmitteltechnologie und angrenzenden Gebieten bekanntermaßen verwendbaren Hilfsstoffe, insbesondere die in einschlägigen Arzneibüchern (z. B. DAB Ph. Eur. BP NF) gelisteten sowie andere Hilfsstoffe, deren Eigenschaften einer physiologischen Anwendung nicht entgegenstehen.

Geeignete Hilfsstoffe können sein: Gleitmittel, Netzmittel, emulgierende und suspendierende Mittel, konservierende Mittel, Antioxidantien, Antireizstoffe, Chelatbildner, Emulsionsstabilisatoren, Filmbildner, Gelbildner, Geruchsmaskierungsmittel, Harze, Hydrokolloide, Lösemittel, Lösungsvermittler, Neutralisierungsmittel, Permeationsbeschleuniger, Pigmente, quaternäre Ammoniumverbindungen, Rückfettungs- und Überfettungsmittel, Salben-, Creme- oder Öl-Grundstoffe, Siliconderivate, Stabilisatoren, Sterilantien, Treibmittel, Trocknungsmittel, Trübungsmittel, Verdickungsmittel, Wachse, Weichmacher, Weißöle. Eine diesbezügliche Ausgestaltung beruht auf fachmännischem Wissen, wie sie beispielsweise in Fiedler, H. P. Lexikon der Hilfsstoffe für Pharmazie, Kosmetik und angrenzende Gebiete, 4. Aufl., Aulendorf: ECV-Editio-Kantor-Verlag, 1996, dargestellt sind.

Zur Herstellung der erfindungsgemäßen dermatologischen Mittel können die Wirkstoffe mit einem geeigneten Hilfsstoff (Exzipient) vermischt oder verdünnt werden. Exzipienten können feste, halb feste oder flüssige Materialien sein, die als Vehikel, Träger oder Medium für den Wirkstoff dienen können. Die Zumischung weiterer Hilfsstoffe erfolgt gewünschtenfalls in der dem Fachmann bekannten Weise.

Nach einer ersten bevorzugten Ausführungsform handelt es sich bei den erfindungsgemäßen Mitteln um ein Hautreinigungsmittel.

Bevorzugte Hautreinigungsmittel sind Seifen von flüssiger bis gelförmiger Konsistenz, wie Transparentseifen, Luxusseifen, Deoseifen, Cremeseifen, Babyseifen, Hautschutzseifen, Abrasiveseifen und Syndets, pasteuse Seifen, Schmierseifen und Waschpasten, flüssige Wasch-, Dusch- und Badepräparate, wie Waschlotionen, Duschbäder und -gele, Schaumbäder, Ölbäder und Scrub-Präparate.

Nach einer weiteren bevorzugten Ausführungsform handelt es sich bei den erfindungsgemäßen Mitteln um kosmetische Mittel zur Pflege und zum Schutz der Haut, Nagelpflegemittel oder Zubereitungen für die dekorative Kosmetik.

Besonders bevorzugt handelt es sich um Hautpflegemittel, Intimpflegemittel, Fußpflegemittel, Lichtschutzmittel, Repellents, Rasiermittel, Haarentfernungsmittel, Antiaknemittel, Make-ups, Mascara, Lippenstifte, Lidschatten, Kajalstifte, Eyeliner, Rouges und Augenbrauenstifte.

Bei den erfindungsgemäßen Hautpflegemitteln handelt es sich insbesondere um W/O- oder O/W-Hautcremes, Tag- und Nachtcremes, Augencremes, Gesichtscremes, Antifaltencremes, Feuchthaltecremes, Bleichcremes, Vitamincremes, Hautlotionen, Pflegelotionen und Feuchthaltelotionen.

Hautkosmetische und dermatologische Mittel auf Basis der zuvor beschriebenen Polymere A) zeigen vorteilhafte Wirkungen. Die Polymere können unter anderem zur Feuchthaltung und Konditionierung der Haut und zur Verbesserung des Hautgefühls beitragen. Die Polymere können auch als Verdicker in den Formulierungen wirken. Durch Zusatz der erfindungsgemäßen Polymere kann in bestimmten Formulierungen eine erhebliche Verbesserung der Hautverträglichkeit erreicht werden.

Hautkosmetische und dermatologische Mittel enthalten vorzugsweise wenigstens ein Copolymer A) in einem Anteil von etwa 0,001 bis 30 Gew.-%, vorzugsweise 0,01 bis 20 Gew.-%, ganz besonders bevorzugt 0,1 bis 12 Gew.-%, bezogen auf das Gesamtgewicht des Mittels.

Besonders Lichtschutzmittel auf Basis der Copolymere A) besitzen die Eigenschaft, die Verweilzeit der UV-absorbierenden Inhaltsstoffe im Vergleich zu gängigen Hilfsmitteln wie Polyvinylpyrrolidon zu erhöhen.

Je nach Anwendungsgebiet können die erfindungsgemäßen Mittel in einer zur Hautpflege geeigneten Form, wie z. B. als Creme, Schaum, Gel, Stift, Mousse, Milch, Spray (Pumpspray oder treibmittelhaltiger Spray) oder Lotion appliziert werden.

Die hautkosmetischen Zubereitungen können neben den Polymeren A) und geeigneten Trägern noch weitere in der Hautkosmetik übliche Wirkstoffe und Hilfsstoffe, wie zuvor beschrieben, enthalten. Dazu zählen vorzugsweise Emulgatoren, Konservierungsmittel, Parfümöle, kosmetische Wirkstoffe wie Phytantriol, Vitamin A, E und C, Retinol, Bisabolol, Panthenol, Lichtschutzmittel, Bleichmittel, Färbemittel, Tönungsmittel, Bräunungsmittel, Collagen, Eiweißhydrolysate, Stabilisatoren, pH-Wert-Regulatoren, Farbstoffe, Salze, Verdicker, Gelbildner, Konsistenzgeber, Silicone, Feuchthaltemittel, Rückfetter und weitere übliche Additive.

Bevorzugte Öl- und Fettkomponenten der hautkosmetischen und dermatologischen Mittel sind die zuvor genannten mineralischen und synthetischen Öle, wie z. B. Paraffine, Siliconöle und aliphatische Kohlenwasserstoffe mit mehr als 8 Kohlenstoffatomen, tierische und pflanzliche Öle, wie z. B. Sonnenblumenöl, Kokosöl, Avocadoöl, Olivenöl, Lanolin, oder Wachse, Fettsäuren, Fettsäureester, wie z. B. Triglyceride von C₆-C₃₀-Fettsäuren, Wachsester, wie z. B. Jojobaöl, Fettalkohole, Vaseline, hydriertes Lanolin und azetyliertes Lanolin sowie Mischungen davon.

Man kann die erfindungsgemäßen Polymere auch mit herkömmlichen Polymeren abmischen, falls spezielle Eigenschaften eingestellt werden sollen.

Zur Einstellung bestimmter Eigenschaften wie z. B. Verbesserung des Anfassgefühls, des Spreitverhaltens, der Wasserresistenz und/oder der Bindung von Wirk- und Hilfsstoffen, wie Pigmenten, können die hautkosmetischen und dermatologischen Zubereitungen zusätzlich auch konditionierende Substanzen auf Basis von Siliconverbindungen enthalten. Geeignete Siliconverbindungen sind beispielsweise Polyalkylsiloxane, Polyarylsiloxane, Polyarylalkylsiloxane, Polyethersiloxane oder Siliconharze.

Die Herstellung der kosmetischen oder dermatologischen Zubereitungen erfolgt nach üblichen, dem Fachmann bekannten Verfahren.

Bevorzugt liegen die kosmetischen und dermatologischen Mittel in Form von Emulsionen insbesondere als Wasser-in-Öl-(W/0)- oder Öl-in-Wasser(O/W)-Emulsionen vor. Es ist aber auch möglich, andere Formulierungsarten zu wählen, beispielsweise Hydrodispersionen, Gele, Öle, Oleogele, multiple Emulsionen, beispielsweise in Form von W/O/W- oder O/W/O-Emulsionen, wasserfreie Salben bzw. Salbengrundlagen, usw.

Die Herstellung von Emulsionen erfolgt nach bekannten Methoden. Die Emulsionen enthalten neben dem Copolymer A) in der Regel übliche Bestandteile, wie Fettalkohole, Fettsäureester und insbesondere Fettsäuretriglyceride, Fettsäuren, Lanolin und Derivate davon, natürliche oder synthetische Öle oder Wachse und Emulgatoren in Anwesenheit von Wasser. Die Auswahl der Emulsionstyp-spezifischen Zusätze und die Herstellung geeigneter Emulsionen ist beispielsweise beschrieben in Schrader, Grundlagen und Rezepturen der Kosmetika, Hüthig Buch Verlag, Heidelberg, 2. Auflage, 1989, dritter Teil, worauf hiermit ausdrücklich Bezug genommen wird.

Eine geeignete Emulsion, z. B. für eine Hautcreme etc., enthält im Allgemeinen eine wässrige Phase, die mittels eines geeigneten Emulgatorsystems in einer Öl- oder Fettphase emulgiert ist.

Der Anteil des Emulgatorsystems beträgt in diesem Emulsionstyp bevorzugt etwa 4 und 35 Gew.-%, bezogen auf das Gesamtgewicht der Emulsion. Vorzugsweise beträgt der Anteil der Fettphase etwa 20 bis 60 Gew.-%. Vorzugsweise beträgt der Anteil der wässrigen Phase etwa 20 und 70 %, jeweils bezogen auf das Gesamtgewicht der Emulsion. Bei den Emulgatoren handelt es sich um solche, die in diesem Emulsionstyp üblicherweise verwendet werden. Sie werden z. B. ausgewählt unter: C₁₂-C₁₈-Sorbitan-Fettsäureestern; Estern von Hydroxystearinsäure und C₁₂-C₃₀-Fettalkoholen; Mono- und Diestern von C₁₂-C₁₈-Fettsäuren und Glycerin oder Polyglycerin; Kondensaten von Ethylenoxid und Propylenglykolen; oxypropylenierten/oxyethylierten C₁₂-C₁₈-Fettalkoholen; polycyclischen Alkoholen, wie Sterolen; aliphatischen Alkoholen mit einem hohen Molekulargewicht, wie Lanolin; Mischungen von oxypropylenierten/polyglycerinierten Alkoholen und Magnesiumisostearat; Succinestern von polyoxyethylenierten oder polyoxypropylenierten Fettalkoholen; und Mischungen von Magnesium-, Calcium-, Lithium-, Zink - oder Aluminiumlanolat und hydriertem Lanolin oder Lanolinalkohol.

Bevorzugte Fettkomponenten, welche in der Fettphase der Emulsionen enthalten sein können, sind: Kohlenwasserstofföle, wie Paraffinöl, Purcellinöl, Perhydrosqualen und Lösungen mikrokristalliner Wachse in diesen Ölen; tierische oder pflanzliche Öle, wie Süßmandelöl, Avocadoöl, Calophylumöl, Lanolin und Derivate davon, Ricinusöl, Sesamöl, Olivenöl, Jojobaöl, Karité-Öl, Hoplostethus-Öl; mineralische Öle, deren Destillationsbeginn unter Atmosphärendruck bei ca. 250 °C und deren Destillationsendpunkt bei 410 °C liegt, wie z. B. Vaselinöl; Ester gesättigter oder ungesättigter Fettsäuren, wie Alkylmyristate, z. B. i-Propyl-, Butyl- oder Cetylmyristat, Hexadecylstearat, Ethyl- oder i-Propylpalmitat, Octan- oder Decansäuretriglyceride und Cetylricinoleat.

Die Fettphase kann auch in anderen Ölen lösliche Siliconöle, wie Dimethylpolysiloxan, Methylphenylpolysiloxan und das Siliconglykol-Copolymer, Fettsäuren und Fettalkohole enthalten.

Um die Retention von Ölen zu begünstigen, können neben den Polymeren A) auch Wachse verwendet werden, wie z. B. Carnaubawachs, Candilillawachs, Bienenwachs, mikrokristallines Wachs, Ozokeritwachs und Ca-, Mg- und Al-Oleate, -Myristate, -Linoleate und -Stearate.

Im Allgemeinen werden die Wasser-in-Öl-Emulsionen so hergestellt, dass die Fettphase und der Emulgator in einen Ansatzbehälter gegeben werden. Man erwärmt diesen bei einer Temperatur von etwa 50 bis 75 °C, gibt dann die in Öl löslichen Wirkstoffe und/oder Hilfsstoffe zu und fügt unter Rühren Wasser hinzu, welches vorher etwa auf die gleiche Temperatur erwärmt wurde und worin man gegebenenfalls die wasserlöslichen Ingredienzien vorher gelöst hat. Man rührt, bis man eine Emulsion der gewünschten Feinheit erhält und lässt dann auf Raumtemperatur abkühlen, wobei gegebenenfalls weniger gerührt wird.

Nach einer weiteren bevorzugten Ausführungsform handelt es sich bei den erfindungsgemäßen Mitteln um ein Duschgel, eine Shampoo-Formulierung oder ein Badepräparat.

Solche Formulierungen enthalten wenigstens ein Polymer A) sowie üblicherweise anionische Tenside als Basistenside und amphotere und/oder nichtionische Tenside als Cotenside. Weitere geeignete Wirkstoffe und/oder Hilfsstoffe sind im Allgemeinen ausgewählt unter Lipiden, Parfümölen, Farbstoffen, organischen Säuren, Konservierungsstoffen und Antioxidantien sowie Verdickern/Gelbildnern, Hautkonditioniermitteln und Feuchthaltemitteln.

Diese Formulierungen enthalten vorzugsweise 2 bis 50 Gew.-%, bevorzugt 5 bis 40 Gew.-%, besonders bevorzugt 8 bis 30 Gew.-% Tenside, bezogen auf das Gesamtgewicht der Formulierung.

In den Wasch-, Dusch- und Badepräparaten können alle in Körperreinigungsmitteln üblicherweise eingesetzten anionische, neutrale, amphotere oder kationische Tenside verwendet werden.

Geeignete anionische Tenside sind beispielsweise Alkylsulfate, Alkylethersulfate, Alkylsulfonate, Alkylarylsulfonate, Alkylsuccinate, Alkylsulfosuccinate, N-Alkoylsarkosinate, Acyltaurate, Acylisethionate, Alkylphosphate, Alkyletherphosphate, Alkylethercarboxylate, Alpha-Olefinsulfonate, insbesondere die Alkali- und Erdalkalimetallsalze, z. B. Natrium, Kalium, Magnesium, Calcium, sowie Ammonium- und Triethanolamin-Salze. Die Alkylethersulfate, Alkyletherphosphate und Alkylethercarboxylate können zwischen 1 bis 10 Ethylenoxid- oder Propylenoxideinheiten, bevorzugt 1 bis 3 Ethylenoxideinheiten im Molekül aufweisen.

Dazu zählen z. B. Natriumlaurylsulfat, Ammoniumlaurylsulfat, Natriumlaurylethersulfat, Ammoniumlaurylethersulfat, Natriumlaurylsarkosinat, Natriumoleylsuccinat, Ammoniumlaurylsulfosuccinat, Natriumdodecylbenzolsulfonat, Triethanolamindodecylbenzolsulfonat.

Geeignete amphotere Tenside sind z. B. Alkylbetaine, Alkylamidopropylbetaine, Alkylsulfobetaine, Alkylglycinate, Alkylcarboxyglycinate, Alkylamphoacetate oder -propionate, Alkylamphodiacetate oder -dipropionate.

Beispielsweise können Cocodimethylsulfopropylbetain, Laurylbetain, Cocamidopropylbetain oder Natriumcocamphopropionat eingesetzt werden.

Als nichtionische Tenside sind beispielsweise geeignet die Umsetzungsprodukte von aliphatischen Alkoholen oder Alkylphenolen mit 6 bis 20 C-Atomen in der Alkylkette, die linear oder verzweigt sein kann, mit Ethylenoxid und/oder Propylenoxid. Die Menge Alkylenoxid beträgt ca. 6 bis 60 Mole auf ein Mol Alkohol. Ferner sind Alkylaminoxide, Mono- oder Dialkylalkanolamide, Fettsäureester von Polyethylenglykolen, ethoxylierte Fettsäureamide, Alkylpolyglycoside oder Sorbitanetherester geeignet.

Außerdem können die Wasch-, Dusch- und Badepräparate übliche kationische Tenside enthalten, wie z. B. quaternäre Ammoniumverbindungen, beispielsweise Cetyltrimethylammoniumchlorid.

Zusätzlich können auch weitere übliche kationische Polymere eingesetzt werden, so z. B. Copolymere aus Acrylamid und Dimethyldiallylammoniumchlorid (Polyquaternium-7), kationische Cellulosederivate (Polyquaternium-4, -10), Guarhydroxypropyltrimethylammoniumchlorid (INCI: Hydroxylpropyl Guar Hydroxypropyltrimonium Chloride), Copolymere aus N-Vinylpyrrolidon und quaternisiertem N-Vinylimidazol (Polyquaterinium-16, -44, -46), Copolymere aus N-Vinylpyrrolidon/Dimethylaminoethylmethacrylat, quaternisiert mit Diethylsulfat (Polyquaternium-11) und andere.

Weiterhin können die Duschgel-/Shampoo-Formulierungen Verdicker, wie z. B. Kochsalz, PEG-55, Propylene Glykol Oleate, PEG-120 Methyl Glucose Dioleate und andere, sowie Konservierungsmittel, weitere Wirk- und Hilfsstoffe und Wasser enthalten.

Nach einer weiteren bevorzugten Ausführungsform handelt es sich bei den erfindungsgemäßen Mitteln um ein Haarbehandlungsmittel.

Erfindungsgemäße Haarbehandlungsmittel enthalten vorzugsweise wenigstens ein Copolymer A) in einer Menge im Bereich von etwa 0,1 bis 30 Gew.-%, bevorzugt 0,5 bis 20 Gew.-%, bezogen auf das Gesamtgewicht des Mittels.

Vorzugsweise liegen die erfindungsgemäßen Haarbehandlungsmittel in Form eines Schaumfestigers, Haarwassers, Haarmousses, Haargels, Shampoos, Haarsprays oder Haarschaums vor. Haarsprays umfassen dabei sowohl Aerosolsprays als auch Pumpsprays ohne Treibgas. Haarschäume umfassen sowohl Aerosolschäume wie auch Pumpschäume ohne Treibgas.

Bevorzugte Haarbehandlungsmittel liegen in Form eines Gels vor. Ein solches Haarbehandlungsmittel enthält beispielsweise:
a) 0,1 bis 20 Gew.-%, bevorzugt 1 bis 10 Gew.-%, mindestens eines Polymers A), wie zuvor definiert,
b) 0 bis 40 Gew.-% wenigstens eines Trägers (Lösungsmittels), der ausgewählt ist unter C₂-C₅-Alkoholen, insbesondere Ethanol,
c) 0,01 bis 5 Gew.-%, bevorzugt 0,2 bis 3 Gew.-%, wenigstens eines Verdickers,
d) 0 bis 10 Gew.-%, bevorzugt 0,1 bis 3 Gew.-%, mindestens eines von a) verschiedenen Festigerpolymers, vorzugsweise eines wasserlöslichen nichtionischen Polymers,
e) 0 bis 1 Gew.-% wenigstens eines Rückfetters, vorzugsweise ausgewählt unter Glycerin und Glycerinderivaten,
f) 0 bis 1 Gew.-% weiterer Wirk- und/oder Hilfsstoffe, z. B. wenigstens eine Siliconverbindung,
g) 0 bis 1 Gew.-% wenigstens eines UV-Absorbers,
h) Wasser ad 100 Gew.-%.

Die Haarbehandlungsmittel können weiterhin in Form von Lösungen (forming water oder styling water) Haarsprays oder Haarschäumen vorliegen. Haarsprays und Haarschäume umfassen vorzugsweise überwiegend oder ausschließlich wasserlösliche oder wasserdispergierbare Komponenten. Sind die in den erfindungsgemäßen Haarsprays und Haarschäumen eingesetzten Verbindungen wasserdispergierbar, können sie in Form von wässrigen Mikrodispersionen mit Teilchendurchmessern von üblicherweise 1 bis 350 nm, bevorzugt 1 bis 250 nm, zur Anwendung gebracht werden. Die Feststoffgehalte dieser Präparate liegen dabei üblicherweise in einem Bereich von etwa 0,5 bis 20 Gew.-%. Diese Mikrodispersionen benötigen in der Regel keine Emulgatoren oder Tenside zu ihrer Stabilisierung.

Weiter können die erfindungsgemäßen Haarbehandlungsmittel im Allgemeinen übliche kosmetische Hilfsstoffe enthalten, beispielsweise Weichmacher, wie Glycerin und Glykol; Emollienzien; Parfüms; Tenside; UV-Absorber; Farbstoffe; antistatische Mittel; Mittel zur Verbesserung der Kämmbarkeit; Konservierungsmittel; und Entschäumer.

Wenn die erfindungsgemäßen Mittel als Haarspray formuliert sind, enthalten sie eine ausreichende Menge eines Treibmittels, beispielsweise einen niedrigsiedenden Kohlenwasserstoff oder Ether, wie Propan, Butan, Isobutan oder Dimethylether. Als Treibmittel sind auch komprimierte Gase brauchbar, wie Stickstoff, Luft oder Kohlendioxid. Die Menge an Treibmittel kann dabei gering gehalten werden, um den VOC-Gehalt nicht unnötig zu erhöhen. Sie beträgt dann im Allgemeinen nicht mehr als 55 Gew.-%, bezogen auf das Gesamtgewicht des Mittels. Gewünschtenfalls sind aber auch höhere VOC-Gehalte von 85 Gew.-% und darüber möglich.

Die zuvor beschriebenen Polymere A) können auch in Kombination mit anderen Haarpolymeren in den Mitteln zur Anwendung kommen. Geeignete Polymere sind die zuvor beschriebenen.

Die anderen Haarpolymere sind vorzugsweise in Mengen bis zu 10 Gew.-%, bezogen auf das Gesamtgewicht des Mittels enthalten.

Ein bevorzugtes Haarbehandlungsmittel in Form eines Haarsprays oder Haarschaums enthält:
a) 0,5 bis 20 Gew.-%, bevorzugt 1 bis 10 Gew.-%, mindestens eines Polymers A), wie zuvor definiert,
b) 50 bis 99,5 Gew.-%, bevorzugt 55 bis 99 Gew.-%, eines Trägers (Lösungsmittels), ausgewählt unter Wasser und wassermischbaren Lösungsmitteln, bevorzugt C₂-C₅-Alkoholen, insbesondere Ethanol, und Mischungen davon,
c) 3 bis 70 Gew.-%, bevorzugt 5 bis 50 Gew.-%, eines Treibmittels, vorzugsweise ausgewählt unter Dimethylether und Alkanen, wie z. B. Propan/Butan-Gemischen,
d) 0 bis 10 Gew.-%, bevorzugt 0,1 bis 10 Gew.-%, mindestens eines von a) verschiedenen Haarpolymers, vorzugsweise eines in Wasser löslichen oder dispergierbaren Polymers,
e) 0 bis 0,5 Gew.-%, bevorzugt 0,001 bis 2 Gew.-%, mindestens einer wasserlöslichen oder wasserdispergierbaren Siliconverbindung,
sowie gegebenenfalls weitere Wirkstoffe und/oder Hilfsstoffe, wie zuvor definiert.

Das erfindungsgemäße Mittel kann als Komponente e) mindestens ein nichtionisches, siloxanhaltiges, wasserlösliches oder -dispergierbares Polymer, insbesondere ausgewählt unter den zuvor beschriebenen Polyethersiloxanen, enthalten. Der Anteil dieser Komponente beträgt dann im Allgemeinen etwa 0,001 bis 2 Gew.-%, bezogen auf das Gesamtgewicht des Mittels.

Die Copolymere A) eignen sich in vorteilhafter Weise zur Einstellung der rheologischen Eigenschaften von Formulierungen, die diese enthalten. Sie wirken dabei als Verdicker, so dass auf den Einsatz zusätzlicher verdickender Substanzen oftmals verzichtet werden kann oder deren Einsatzmenge zumindest verringert werden kann.

Die Copolymere A) eignen sich in vorteilhafter Weise als Hilfsmittel in der Pharmazie, z. B. als Tablettenüberzugsmittel, Bindemittel oder Umhüllung von Kapseln, als oder in Beschichtungsmittel(n) für die Textil-, Papier-, Druck- und Lederindustrie sowie für Agrochemikalien.

Ein weiterer Gegenstand der vorliegenden Erfindung ist ein Verfahren zur Herstellung eines wie vorstehend definierten Copolymeren A durch radikalische Polymerisation der Monomere a) mit wenigstens einem weiteren Monomer ausgewählt unter den Monomeren b) und c) gegebenenfalls in Gegenwart von bis zu 25 Gew.-%, bezogen auf das Gesamtgewicht der Komponenten a) bis d), einer wasserlöslichen Komponente d), dadurch gekennzeichnet, dass man die Polymerisation in einem wässrigen Lösungsmittel durchführt. Die obigen Ausführungen zu den bevorzugten Ausgestaltungen der Polymerisation zur Herstellung des erfindungsgemäßen Copolymers A gelten hier entsprechend.

### Beispiele

### 1. Herstellung von Polyallylpolyetherurethanen

### I) Mono-/Diallylpolyetherurethan

### Beispiel A:

### Einsatzmaterial:

500 g (= 1 mol) Pluriol® A 010R (Fa. BASF Aktienges.)
83 g (= 0,5 mol) Hexamethylendiisocyanat

In einem Vierhalskolben, der mit Rührer, Tropftrichter, Thermometer, Rückflusskühler und einer Vorrichtung für das Arbeiten unter Stickstoff ausgestattet war, wurden 500 g (1 mol) Pluriol® A 010R und 0,1 g Tetrabutylorthotitanat in 150 g Methylethylketon unter Erhitzen auf eine Temperatur von ca. 50 °C und unter Rühren gelöst. Anschließend wurden unter Rühren 83 g (0,5 mol) Hexamethylendiisocyanat zugetropft, wobei die Reaktionstemperatur anstieg. Unter Rückfluss wurde das Reaktionsgemisch etwa 2 h gerührt. Man verdünnte das Reaktionsgemisch mit 435 g Ethanol und kühlte danach auf Raumtemperatur ab. Es wurde eine klare 50 gew.-%ige Polyallylpolyetherurethan-Lösung erhalten.

Analog wurden die Polyallylpolyetherurethan-Oligomere B bis F hergestellt. Des Weiteren können auch die Allylpolyetherurethane L bis O (für die im Folgenden lösungsmittelfreie oder lösungsmittelarme Herstellungsverfahren angegeben werden) nach dieser Methode hergestellt werden.

### II) Dimethylsiloxan-haltiges Polyallylpolyetherurethan

### Beispiel G:

### Einsatzmaterial:

50 g (= 0,1 mol) Pluriol® A 010R (Fa. BASF Aktienges.)
22,2 g (= 0,1 mol) Isophorondiisocyanat ,
133 g (= 0,038 mol) Pluriol® ST 4005 (Fa. BASF Aktienges.)

In einem Vierhalskolben, der mit Rührer, Tropftrichter, Thermometer, Rückflusskühler und einer Vorrichtung für das Arbeiten unter Stickstoff ausgestattet war, wurden 50 g (0,1 mol) Pluriol® A 010R und 0,03 g Tetrabutylorthotitanat in 15 g Methylethylketon unter Erwärmen auf eine Temperatur von ca. 50 °C und unter Rühren vorgelegt. Anschließend wurden unter Rühren 22,2 g (0,1 mol) Isophorondiisocyanat zugetropft, wobei die Reaktionstemperatur anstieg. Nach 20 Minuten Rühren bei 80 °C wurden 133 g (= 0,038 mol) Pluriol® ST 4005 zugegeben. Das Reaktionsgemisch wurde noch 3 h bei 80 °C gerührt, dann mit 190 g Ethanol versetzt und weitere 30 Minuten bei 80 °C gerührt. Nach Abkühlen auf Raumtemperatur erhielt man eine klare 50 gew.-%ige Polyallylpolyetherurethan-Lösung.

Analog wurden die Polyallylpolyurethane H bis K hergestellt.

**Tabelle 1: Polyallylpolyetherurethane**

| | Pluriol® A 010R | Pluriol® A 011R | Pluriol® ST 4005 | Tegomer H-Si2111 | NPG | IPDI | HDI | t-BAEMA |
|---|---|---|---|---|---|---|---|---|
| | [mol%] | [mol%] | [mol%]* | [mol%] | [mol%] | [mol%] | [mol%] | [mol%] |
| A | 66,67 | -- | -- | -- | -- | -- | 33,33 | -- |
| B | 57,14 | -- | -- | -- | 7,14 | -- | 35,72 | -- |
| C | 62,07 | -- | -- | 1,72 | 1,72 | -- | 34,49 | -- |
| D | -- | 54,23 | -- | 1,70 | -- | -- | 33,90 | 10,17 |
| E | -- | 66,67 | -- | -- | -- | 33,33 | -- | -- |
| F | 57,14 | -- | -- | 7,14 | -- | 35,72 | -- | -- |
| G | 42,02 | -- | 15,96 | -- | -- | 42,02 | -- | -- |
| H | -- | 42,02 | 15,96 | -- | -- | 42,02 | -- | -- |
| I | 42,02 | -- | 15,96 | -- | -- | 21,01 | 21,01 | -- |
| K | -- | 38,76 | 14,73 | -- | 7,75 | 38,76 | -- | -- |

| | | | | | | | | |
|---|---|---|---|---|---|---|---|---|
| Pluriol® A 010R: Allylalkoholethoxilat, M_{w} = ca. 500, Fa. BASF Aktienges. Pluriol® A 011R: Allylalkoholethoxilatpropoxilat, M_{w} = ca. 2000, Fa. BASF Aktienges. Pluriol® ST 4005: ethoxiliertes propoxiliertes Polydimethylsiloxan, M_{w} = ca. 8000, Fa. BASF Aktienges. *) M_{w} ca. 3500 (umgerechnet für Silikon mit zwei OH-Gruppen) Tegomer H-Si 2111: Poly(dimethylsiloxan)diol, M_{w} = ca. 900, Fa. Goldschmidt NPG: Neopentylglykol IPDI: Isophorondiisocyanat HDI: Hexamethylendiisocyanat t-BAEMA: tert.-Butylaminoethylmethacrylat | | | | | | | | |

### III)Allylgruppen-haltige Polyetherurethane

### Beispiel L:

Die Herstellung erfolgte in der bei Beispiel A beschriebenen Apparatur lösungsmittelfrei in einem einstufigen Verfahren.

### Beispiel M:

Zweistufiges Verfahren, Stufe 1 lösungsmittelfrei, Stufe 2 in Ethanol.

In einer Apparatur, wie in Beispiel A beschrieben, wurden 57 % der insgesamt eingesetzten Polyethylenglykolmenge, das Pluriol® A 010R sowie das Isophorondiisocyanat lösungsmittelfrei unter Erwärmen auf eine Temperatur von ca. 80 °C zu einem isocyanatgruppenhaltigen Präpolymer umgesetzt. Nach Abkühlen auf etwa 40 °C wurde mit Ethanol versetzt und das Präpolymer mit der Restmenge des Polyethylenglykols, dem Tegomer A-Si 2122 sowie dem Hexamethylendiisocyanat zum Endprodukt umgesetzt.

### Beispiel N:

Zweistufiges Herstellungsverfahren, Pluriol® A 010R und IPDI in der ersten Stufe, Pluriol® ST 4005 in der zweiten Stufe, beide Stufen lösungsmittelfrei.

### Beispiel O:

zweistufiges Verfahren, Polyethylenglykol, Pluriol® A 010R und IPDI in der ersten Stufe, Pluriol® ST 4005 in der zweiten Stufe, beide Stufen lösungsmittelfrei.

**Tabelle 2: Allylgruppen-haltige Polyetherurethane**

| | PEG 1000 | Pluriol® A 010R | Tegomer H-Si 2122 | Tegomer A-Si 2122 | Pluriol® ST 4005 | IPDI | HDI |
|---|---|---|---|---|---|---|---|
| | [mol%] | [mol%] | [mol%] | [mol%] | [mol%] | [mol%] | [mol%] |
| L | 10 | 40 | 10 | | | 40 | |
| M | 31,82 | 18,18 | | 4,55 | | 31,82 | 13,63 |
| N | | 38,46 | | | 23,08 | 38,46 | |
| O | 21,74 | 17,39 | | | 17,39 | 43,48 | |

| | | | | | | | |
|---|---|---|---|---|---|---|---|
| PEG 1000: Polyethylenglykol, M_{w} = 1000 Tegomer H-Si 2122: Poly(dimethylsiloxan)diol, M_{w} = ca. 1000 Tegomer A-Si 2122: Poly(dimethylsiloxan)diamin, M_{w} = ca. 1000 | | | | | | | |

### 2. Herstellung von Copolymerisaten (Lösungspolymerisation)

### Beispiel 6: Copolymer aus VP/MAM/VI/Polyallylpolyetherurethan A

Zulauf 1: Monomerengemisch aus:

| | |
|---|---|
| 342 g | Vinylpyrrolidon |
| 12 g | Vinylimidazol |
| 1400 g | einer 15%igen wässrigen Lösung von Methacrylamid (= 210 g Methacrylamid und 1190 g Wasser) |
| 7,2 g | einer 50%igen ethanolischen Polyallylpoly- |
| | etherurethan-Lösung |
| 3,5 g | 85%ige wässrige Phosphorsäure (ca. 3 g H₃PO₄) |

Zulauf 2: Initiatorlösung aus:

| | |
|---|---|
| 6 g | Wako V 50 [2,2'-Azobis(2-amidinopropan)-dihydrochlorid] und |
| 123 g | Wasser |

Zulauf 3: Initiatorlösung aus:

| | |
|---|---|
| 4 g | Wako V 50 [2,2'-Azobis(2-amidinopropan)-dihydrochlorid] und |
| 82 g | Wasser |

In einer Rührapparatur mit Rückflusskühler, Innenthermometer und 4 separaten Zulaufvorrichtungen wurden 176 g von Zulauf 1 und 12,9 g von Zulauf 2 vorgelegt und die Mischung unter Rühren auf ca. 65 °C aufgeheizt. Nach dem Anpolymerisieren, erkennbar an einer beginnenden Viskositätserhöhung, wurde bei 65 °C der Rest von Zulauf 1 innerhalb von 3 h und der Rest von Zulauf 2 in 4 h zugegeben, wobei die Innentemperatur auf ca. 68 °C erhöht wurde. Nach dem Ende der Zugabe wurde das Reaktionsgemisch noch ca. 2 h bei 70 °C gerührt. Anschließend wurde der Zulauf 3 innerhalb von 30 Minuten bei einer Temperatur von 70 °C hinzugefügt und die Polymerlösung anschließend noch ca. 2 h bei einer Temperatur von ca. 80 °C nachpolymerisiert. Die Polymerlösung wurde 2 h mit Wasserdampf behandelt. Man erhielt eine ca. 30%ige wässrige Mikrodispersion.

Zum Stabilisieren wurde die Lösung mit 100 ppm Euxyl K100 der Fa. Schülke & Mayr (5-Chlor-2-methyl-3-(2)-isothiazolon/2-Methyl-3-(2H)-isothiazolon/Benzylalkohol) versetzt.

Pulverförmige Produkte wurden durch Sprühtrocknen oder Gefriertrocknen erhalten.

Analog hierzu wurden alle Produkte, die in der folgenden Tabelle 2 aufgeführt sind, polymerisiert.

| | VP [Gew.%] | MAM [Gew.%] | VFA [Gew.%] | VI [Gew.%] | QVI [Gew.%] | DAD-MAC [Gew.%] | PAPEU 1 [Gew.%] | PAPEU 2 [Gew.%] | DA WA [Gew.%] | PEG 6000 [Gew.%] |
|---|---|---|---|---|---|---|---|---|---|---|
| 1 | 70 | -- | 25 | -- | -- | -- | 5 | -- | -- | -- |
| 2 | 70 | -- | 22 | -- | -- | -- | 5 | 3 | -- | -- |
| 3 | 70 | 20 | -- | -- | -- | -- | 10 | -- | -- | -- |
| 4 | 60 | 30 | -- | -- | -- | -- | 5 | 5 | -- | -- |
| 5 | 60 | 35 | -- | | -- | -- | -- | 5 | -- | -- |
| 6 | 57 | 35 | -- | 2 | -- | -- | 6 | -- | -- | -- |
| 7 | 57 | 35 | -- | 3 | -- | -- | -- | 5 | -- | -- |
| 8 | 50 | 30 | -- | | 15 | -- | -- | 5 | -- | -- |
| 9 | 40 | 40 | -- | | -- | 15 | -- | 5 | -- | -- |
| 10 | -- | 40 | 40 | | - | 15 | -- | 5 | -- | -- |
| 11 | 50 | -- | 30 | | -- | 15 | -- | 5 | -- | -- |
| 12 | 50 | -- | 30 | | -- | 15 | -- | 5 | -- | -- |
| 13 | 40 | -- | 30 | | -- | 25 | -- | 5 | -- | -- |
| 14 | 35 | -- | 35 | | -- | 20 | -- | 10 | -- | -- |
| 15 | 35 | -- | 35 | | -- | 20 | -- | 9.5 | 0.5 | -- |
| 16 | 35 | -- | 35 | | -- | 20 | -- | 9.7 | 0.3 | -- |
| 17 | 30 | -- | 45 | | -- | 20 | -- | 4.7 | 0.3 | -- |
| 18 | 20 | -- | 55 | | -- | 15 | 5 | 4.7 | 0.3 | -- |
| 19 | -- | -- | 80 | | -- | 10 | -- | 9.5 | 0.5 | -- |
| 20 | -- | -- | 80 | | -- | 10 | -- | 4.5 | 0.5 | 5 |

| | | | | | | | | | | |
|---|---|---|---|---|---|---|---|---|---|---|
| VP = Vinylpyrrolidon MAM = Methacrylsäureamid VFA = Vinylformamid VI = Vinylimidazol QVI = Vinylimidazoliummethylchlorid DADMAC = Diallyldimethylammoniumchlorid PAPEU 1 = Polyallypolyetherurethan aus Allylalkoholethoxilat Pluriol® A 010R, (Fa. BASF) und Hexamethylendiisocyanat (aus Beispiel A) PAPEU 2 = Polyallylpolyetherurethan aus Allylalkoholethoxilat Pluriol® ST 4005, (Fa. BASF) und Isophorondiisocyanat (aus Beispiel G) DAWA = N,N'-Diallylweinsäurediamid PEG 6000 = Polyethylenglykol, Mₙ = 6000 | | | | | | | | | | |

### 3. Anwendungstechnische Beispiele

Verwendung in der Haarkosmetik:

### 1) Haargele mit einem anionischen Verdicker: Beispiele Nr. 21-27

| Phase 1: | [%] | CTFA |
|---|---|---|
| Polymer 1-7 (30%ige wässrige Lösung) | 10,0 | |
| Glycerin | 0,3 | |
| Wasser dest. | 39,2 | |
| Weitere Zusatzstoffe: Konservierungs-mittel, lösliches ethoxiliertes Silikon, Parfüm | q.s. | |

| Phase 2: | | |
|---|---|---|
| Carbopol 940 (1%ige wässrige Suspension) | 30,0 | Carbomer |
| Triethanolamin | 0,5 | |
| Wasser dest. | 20,0 | |

Zur Herstellung von Haargelen werden die Komponenten eingewogen und homogenisiert. Dabei bildet die Phase 2 ein klares, festes Gel, in das Phase 1 langsam eingerührt wird.

### 2) Haargele mit einem weiteren Festigerpolymer und anionischem Verdicker: Beispiele Nr. 28-34

| Phase 1: | [%] | CTFA |
|---|---|---|
| Polymer 1-7 (30%ige wässrige Lösung) | 7,0 | |
| Luviskol VA 64 | 1,0 | Vinylpyrrolidon-vinylacetat-Copolymer |
| Uvinul MS 40 | 0,2 | Benzophenon-4 |
| Glycerin | 0,2 | |
| D-Panthenol USP | 0,1 | Panthenol |
| Ethanol | 20,0 | |
| Wasser dest. | 21,0 | |
| Weitere Zusatzstoffe: Konservierungsmittel, lösliches ethoxyliertes Silikon, Parfüm | q.s. | |

| Phase 2: | | |
|---|---|---|
| Carbopol 940 (1%ige wässrige Suspension) | 30,0 | Carbomer |
| Triethanolamin | 0,5 | |
| Wasser dest. | 20,0 | |

Herstellung: Die Komponenten der beiden Phasen werden nach dem Einwiegen homogenisiert. Phase 2 bildet ein klares, festes Gel. Phase 1 wird langsam in Phase 2 eingerührt.

### 3) Flüssige Haargele: Beispiele Nr. 35-46

| | [%] | CTFA |
|---|---|---|
| Polymer 1-12 (30%ige wässrige Lösung) | 5,3 | |
| Natrosol 250 L (2%ige wässrige Lösung) | 25,0 | Hydroxyethyl-cellulose (Fa. Hercules) |
| C-Dry MD 1915 (10%ige wässrige Lösung) | 25,0 | abgebaute Stärke (Fa. Cerestar) |
| Wasser dest. | 44,7 | |
| Weitere Zusatzstoffe: Konservierungsmittel, lösliches ethoxyliertes Silikon, Parfüm | q.s. | |

Herstellung: Einwiegen und bei Raumtemperatur langsam Homogenisieren.

### 4) Selbstverdickende Haargele (ohne zusätzlichen Verdicker): Beispiele 47-52

| | [%] | CTFA |
|---|---|---|
| Polymer 15-20 (30%ige wässrige Lösung) | 10,0 | |
| Polymer 6 (30%ige wässrige Lösung) | 3,3 | |
| Glycerin | 0,2 | |
| Wasser dest. | 86,5 | |
| Weitere Zusatzstoffe: Konservierungsmittel, lösliches ethoxyliertes Silikon, Parfüm | q.s. | |

Herstellung: Die Komponenten werden eingewogen, bei 40 °C homogenisiert und anschließend unter Rühren auf Raumtemperatur abgekühlt.

### 5) Wässrige Handpumpen-Gele: Beispiele Nr. 53-66

| | [%] | CTFA |
|---|---|---|
| Polymer 1-14 (30%ige wässrige Lösung) | 10,0 | |
| Luviskol®K 90 | 1,0 | Polyvinyl-pyrrolidon (K-Wert 90, Fa. BASF) |
| Wasser dest. | 88,9 | |
| Weitere Zusatzstoffe: Konservierungsmittel, lösliches ethoxyliertes Silikon, Parfüm | q.s. | |

Herstellung: Einwiegen und bei Raumtemperatur langsam Homogenisieren.

### 6) VOC 10 Handpumpen-Spray: Beispiele Nr. 67-73

| | [%] | CTFA |
|---|---|---|
| Polymer 1-7 (30%ige wässrige Lösung) | 10,0 | |
| Belsil®DMC 6031 | 0,10 | ethoxiliertes Silikon (Fa. Goldschmidt) |
| Wasser dest. | 79,89 | |
| Ethanol | 10,0 | |
| Weitere Zusatzstoffe: Konservierungsmittel, lösliches ethoxyliertes Silikon, Parfüm | q.s. | |

### 7) VOC 55 Aerosol-Haarspray: Beispiele Nr. 74-78

| | [%] | CTFA |
|---|---|---|
| Polymer 1-5 (30%ige wässrige Lösung) | 3,0 | |
| Luviset®PUR (30%ige Wasser/Ethanol-Lsg.) | 7,0 | (PU-Dispersion Fa. BASF) |
| Wasser dest. | 35,5 | |
| Dimethylether | 30,0 | |
| Ethanol | 24,5 | |
| Weitere Zusatzstoffe: Konservierungsmittel, lösliches ethoxyliertes Silikon, Parfüm | q.s. | |

Herstellung: Die Polymerkomponenten und Hilfsstoffe werden eingewogen, unter Rühren in Wasser/Ethanol gelöst und anschließend in Sprühbehälter abgefüllt. Abschließend wird das Treibgas zugesetzt.

### 8) Schaumfestiger: Beispiele Nr. 79-90

| | [%] | CTFA |
|---|---|---|
| Polymer 1-12 (30%ige wässrige Lösung) | 5,0 | |
| Cremophor A 25 (Ceteareth 25/BASF) | 0,2 | |
| Comperlan KD (Coamide DEA/Henkel) | 0,1 | |
| Wasser dest. | 84,6 | |
| Dimethylether (3,5 bar, 20 °C) | 10,0 | |
| Weitere Zusatzstoffe: Konservierungsmittel, lösliches ethoxyliertes Silikon, Parfüm | q.s. | |

Herstellung: Einwiegen und unter Rühren lösen. Abfüllen und Treibgas zusetzen.

### 9) Wetlook-Schaumfestiger: Beispiele 91-102

| | [%] | CTFA |
|---|---|---|
| Polymer 1-12 (30%ige wässrige Lösung) | 10,0 | |
| Luviquat® Mono LS | 2,0 | Cocotrimonium Methosulfat |
| Glycerin 87 % | 5,0 | Glycerin |
| Wasser dem. | 72,8 | |
| Parfumöl | 0,2 | |
| Propan/Butan (3,5 bar, 20 °C) | 10,0 | |
| Konservierungsmittel | q. s. | |

Herstellung: Einwiegen und unter Rühren lösen. Abfüllen und Treibgas zusetzen.

### 10) Shampoo: Beispiele Nr. 103-108

### Conditioner Shampoo:

| | [%] | CTFA |
|---|---|---|
| A) Texapon NSO 28%ig (Natriumlauryl-sulfat/Henkel) | 50,0 | |
| Comperlan KD (Coamide DEA/Henkel) | 1,0 | |
| Polymer 15-20 (30%ige wässrige Lösung) | 3,0 | |
| Wasser dest. | 17,0 | |
| Parfümöl | q.s. | |
| B) Wasser | 27,5 | |
| Natriumchlorid | 1,5 | |
| Konservierungsmittel | q.s. | |

Herstellung: Einwiegen und unter Rühren Phasen A) und B) getrennt lösen und mischen. Phase B) langsam in Phase A) einrühren.

### Verwendung in der Hautkosmetik:

### 11) Standard O/W-Creme: Beispiele Nr. 109-120

| Ölphase: | [%] | CTFA |
|---|---|---|
| Cremophor A6 | 3,5 | Ceteareth-6 und Stearylalkohol |
| Cremophor A25 | 3,5 | Ceteareth-25 |
| Glycerinmonostearat s.e. | 2,5 | Glyceryl Stearate |
| Paraffinöl | 7,5 | Paraffin Oil |
| Cetylalkohol | 2,5 | Cetyl Alkohol |
| Luvitol EHO | 3,2 | Cetearyloctanoat |
| Vitamin-E-acetate | 1,0 | Tocopheryl Acetate |
| Nip-Nip | 0,1 | Methyl- und Propyl-4-hydroxybenzoate (7:3) |

| Wasserphase: | [%] | |
|---|---|---|
| Polymer 9-20 (30%ige wässrige Lösung) | 3,0 | |
| Wasser | 74,6 | |
| 1,2-Propylenglycol | 1,5 | |
| Germall II | 0,1 | Imidazolidinyl-Urea |

Herstellung: Einwiegen und unter Rühren die Ölphase und die Wasserphase getrennt bei einer Temperatur von 80 °C homogenisieren. Die Wasserphase langsam in die Ölphase einrühren. Unter Rühren langsam auf Raumtemperatur abkühlen.

### 12) Tageslotion: Beispiele Nr. 121-132

| Ölphase: | [%] | CTFA |
|---|---|---|
| Cremophor A6 | 1,5 | Ceteareth-6 und Stearylalkohol |
| Cremophor A25 | 1,5 | Ceteareth-25 |
| Glycerinmonostearat s.e. | 5,0 | Glyceryl Stearate |
| Uvinul MS 40 | 0,5 | Benzophenone-4 |
| Paraffinöl | 3,5 | Paraffin Oil |
| Cetylalkohol | 0,5 | Cetyl Alkohol |
| Luvitol EHO | 10,0 | Cetearyl Octanoat |
| D-Panthenol 50P | 3,0 | Panthenol und Propylenglycol |
| Vitamin-E-acetate | 1,0 | Tocopheryl Acetate |
| Tegiloxan 100 | 0,3 | Dimethicon |
| Nip-Nip | 0,1 | Methyl- und Propyl-4-hydroxybenzoate (7:3) |

| Wasserphase: | [%] | |
|---|---|---|
| Polymer 9-20 (30%ige wässrige Lösung) | 1,5 | |
| Wasser | 70,0 | |
| 1,2-Propylenglycol | 1,5 | |
| Germall II | 0,1 | Imidazolidinyl-Harnstoff |

Herstellung: Einwiegen und unter Rühren die Ölphase und die Wasserphase getrennt bei einer Temperatur von 80 °C homogenisieren. Die Wasserphase langsam in die Ölphase einrühren. Unter Rühren langsam auf Raumtemperatur abkühlen.

## Patentansprüche

1. Polyetherurethan, enthaltend wenigstens eine Allylgruppe, das
a) wenigstens einen Polyether, der eine gegenüber Isocyanatgruppen reaktive Gruppe und eine Allylgruppe enthält,
b) wenigstens eine Verbindung, die wenigstens zwei gegenüber Isocyanatgruppen reaktive Gruppen enthält, die ausgewählt ist unter Verbindungen mit einem zahlenmittleren Molekulargewicht von mehr als 280, die mindestens zwei aktive Wasserstoffatome und mindestens eine Siloxangruppe pro Molekül enthalten, und
c) wenigstens ein Polyisocyanat,
eingebaut enthält.

2. Polyetherurethan nach Anspruch 1, das wenigstens eine Verbindung b) eingebaut enthält, die ausgewählt ist unter:
- Polysiloxanen der allgemeinen Formel I.1 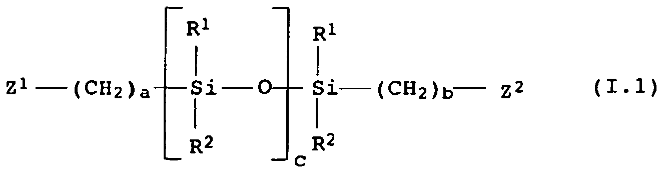 worin
a und b unabhängig voneinander für 1 bis 8 stehen,
c für 2 bis 100 steht,
R¹ und R² unabhängig voneinander für C₁-C₈-Alkyl, Benzyl oder Phenyl stehen,
Z¹ und Z² unabhängig voneinander für OH, NHR³ oder einen Rest der Formel II
-(CH₂CH₂O)ᵥ(CH₂CH(CH₃)O)_{w}-H (II)
stehen, wobei
in der Formel II die Reihenfolge der Alkylenoxideinheiten beliebig ist und
v und w unabhängig voneinander für eine ganze Zahl von 0 bis 200 stehen, wobei die Summe aus v und w > 0 ist,
R³ für Wasserstoff, C₁-C₈-Alkyl oder C₅-C₈-Cycloalkyl steht;
- Polysiloxanen der allgemeinen Formel I.2 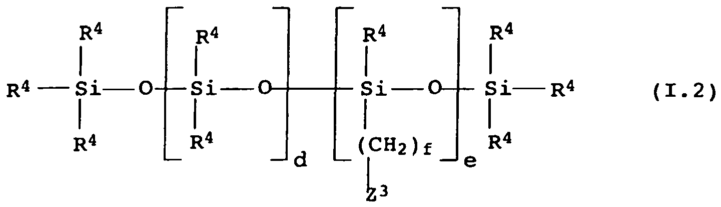 worin
die Reihenfolge der Siloxaneinheiten beliebig ist,
die Reste R⁴ jeweils unabhängig voneinander für C₁-C₈-Alkyl, vorzugsweise Methyl, Benzyl oder Phenyl stehen,
d für eine ganze Zahl von 5 bis 1000 steht,
e für eine ganze Zahl von 2 bis 100 steht,
f für eine ganze Zahl von 2 bis 8 steht,
Z³ für OH, NHR³, wobei R³ wie zuvor definiert ist, oder einen Rest der Formel III
-(OCH₂CH₂)ₓ(OCH(CH₃)CH₂)_{y}-OH
steht, wobei
in der Formel III die Reihenfolge der Alkylenoxideinheiten beliebig ist,
x und y unabhängig voneinander für eine ganze Zahl von 0 bis 200 stehen, wobei die Summe aus x und y > 0 ist,
und Mischungen davon.

3. Wasserlösliches oder wasserdispergierbares Polymer, das wenigstens ein Polyetherurethan, wie in einem der Ansprüche 1 oder 2 definiert, und wenigstens eine radikalisch polymerisierbare Verbindung, die wenigstens eine α,β-ethylenisch ungesättigte Doppelbindung aufweist, einpolymerisiert enthält.

4. Polymer nach Anspruch 3, das wenigstens eine radikalisch polymerisierbare hydrophile nichtionische Verbindung M1) einpolymerisiert enthält.

5. Polymer nach Anspruch 4, wobei die Verbindung M1) ausgewählt ist unter primären Amiden α,β-ethylenisch ungesättigter Monocarbonsäuren, N-Vinyllactamen, N-Vinylamiden gesättigter Monocarbonsäuren, Estern α,β-ethylenisch ungesättigter Mono - und Dicarbonsäuren mit C₂-C₄-Alkandiolen, Amiden α,β-ethylenisch ungesättigter Mono- und Dicarbonsäuren mit C₂-C₄-Aminoalkoholen, die eine primäre oder sekundäre Aminogruppe aufweisen, Vinylethern, nichtionischen, hydrophilen vinyl- und allylsubstituierten heterocyclischen Verbindungen und Mischungen davon.

6. Polymer nach Anspruch 5, das eine Verbindung M1), die ausgewählt ist unter Acrylsäureamid, Methacrylsäureamid, N-Vinylpyrrolidon, N-Vinylcaprolactam, N-Vinylformamid, N-Vinylacetamid und Mischungen davon einpolymerisiert enthält.

7. Polymer nach einem der Ansprüche 4 bis 6, das zusätzlich wenigstens eine radikalisch polymerisierbare Verbindung M2) mit einer α,β-ethylenisch ungesättigten Doppelbindung und mindestens einer ionogenen und/oder ionischen Gruppe pro Molekül einpolymerisiert enthält.

8. Polymer nach einem der Ansprüche 4 bis 7, das zusätzlich wenigstens eine radikalisch polymerisierbare vernetzende Verbindung mit wenigstens zwei α,β-ethylenisch ungesättigten Doppelbindungen pro Molekül einpolymerisiert enthält.

9. Polymer nach einem der Ansprüche 4 bis 8, das erhältlich ist durch radikalische Copolymerisation in Gegenwart einer Komponente d), die ausgewählt ist unter
d1) polyetherhaltigen Verbindungen,
d2) Polymeren, die mindestens 50 Gew.-% Wiederholungseinheiten aufweisen, die sich von Vinylalkohol ableiten,
d3) Stärke und Stärkederivaten,
und Mischungen davon.

10. Polymer nach einem der Ansprüche 4 bis 9, erhältlich durch radikalische Polymerisation von
- 1 bis 25 Gew.-%, bezogen auf das Gesamtgewicht der zur Polymerisation eingesetzten Komponenten, wenigstens eines Polyallylpolyetherurethans,
- 50 bis 99 Gew.-% wenigstens einer radikalisch polymerisierbaren nichtionischen Verbindung M1),
- 0 bis 25 Gew.-% wenigstens eines Monomers M2) mit mindestens einer ionogenen und/oder ionischen Gruppe pro Molekül,
- 0 bis 10 Gew.-% wenigstens eines Vernetzers,
gegebenenfalls in Gegenwart von bis zu 25 Gew.-% wenigstens einer Komponente d), wie in Anspruch 10 definiert.

11. Verfahren zur Herstellung eines Polymers, wie in einem der Ansprüche 4 bis 10 definiert, durch radikalische Polymerisation in einem wässrigen Lösungsmittel bei einem pH-Wert von 5,5 bis 8,0.

12. Verfahren nach Anspruch 11, umfassend einen ersten Polymerisationsschritt und einen daran anschließenden zweiten Polymerisationsschritt, wobei das Reaktionsgemisch zwischen dem ersten und dem zweiten Polymerisationsschritt einem Strippen mit wasserdampf oder einer Wasserdampfdestillation unterzogen wird.

13. Kosmetisches oder pharmazeutisches Mittel, enthaltend
A) wenigstens ein wasserlösliches oder wasserdispergierbares Polymer, wie in einem der Ansprüche 3 bis 10 definiert, und
B) wenigstens einen kosmetisch akzeptablen Träger.

14. Mittel nach Anspruch 13, wobei die Komponente B) ausgewählt ist unter
i) Wasser,
ii) wassermischbaren organischen Lösungsmitteln, vorzugsweise C₁-C₄-Alkanolen,
iii) Ölen, Fetten, Wachsen,
iv) von iii) verschiedenen Estern von C₆-C₃₀-Monocarbonsäuren mit ein-, zwei- oder dreiwertigen Alkoholen,
v) gesättigten acyclischen und cyclischen Kohlenwasserstoffen,
vi) Fettsäuren,
vi) Fettalkoholen
und Mischungen davon.

15. Mittel nach einem der Ansprüche 13 oder 14, enthaltend außerdem wenigstens einen von Copolymer A verschiedenen Bestandteil, der ausgewählt ist unter kosmetisch aktiven Wirkstoffen, Emulgatoren, Tensiden, Konservierungsmitteln, Parfümölen, Verdickern, Haarpolymeren, Haar- und Hautconditionern, Pfropfpolymeren, wasserlöslichen oder dispergierbaren silikonhaltigen Polymeren, Lichtschutzmitteln, Bleichmitteln, Gelbildnern, Pflegemitteln, Färbemitteln, Tönungsmitteln, Bräunungsmitteln, Farbstoffen, Pigmenten, Konsistenzgebern, Feuchthaltemitteln, Rückfettern, Collagen, Eiweißhydrolysaten, Lipiden, Antioxidantien, Entschäumern, Antistatika, Emollienzien und Weichmachern.

16. Mittel nach einem der Ansprüche 13 bis 15 in Form einer Lösung, eines Gels, Wachses, Schaums, Sprays, einer Salbe, Creme, Emulsion, Suspension, Lotion, Milch oder Paste.

17. Verwendung eines Polymers, wie in einem der Ansprüche 3 bis 10 definiert, in Hautreinigungsmitteln, Mitteln zur Pflege und zum Schutz der Haut, Nagelpflegemitteln, Zubereitungen für die dekorative Kosmetik und Haarbehandlungsmitteln.

18. Verwendung nach Anspruch 17 in Haarbehandlungsmitteln als Verdicker, Festiger und/oder als Conditioner.

19. Verwendung nach Anspruch 18, wobei das Mittel in Form eines Haargels, Haarmousses, Shampoos, Schaumfestigers, Haarwassers, Haarsprays oder Haarschaums vorliegt.

20. Verwendung eines Polymers, wie in einem der Ansprüche 3 bis 10 definiert, als Hilfsmittel in der Pharmazie, bevorzugt als oder in Beschichtungsmittel(n) für feste Arzneiformen sowie als oder in Beschichtungsmittel(n) für die Textil-, Papier-, Druck- und Lederindustrie sowie für die Agrochemie.

## Claims

1. A polyether-urethane comprising at least one allyl group, which comprises, in incorporated form,
a) at least one polyether which comprises a group reactive toward isocyanate groups, and an allyl group,
b) at least one compound which comprises at least two groups reactive toward isocyanate groups, chosen from compounds with a number-average molecular weight of more than 280 which comprise at least two active hydrogen atoms and at least one siloxane group per molecule, and
c) at least one polyisocyanate.

2. The polyether-urethane according to claim 1, which comprises, in incorporated form, at least one compound b) chosen from:
- polysiloxanes of the formula I.1 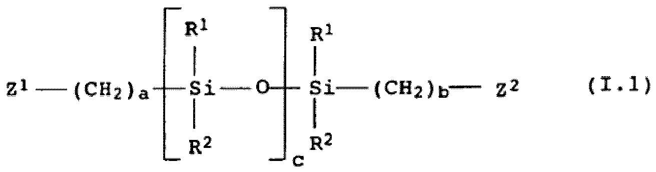 in which
a and b, independently of one another, are 1 to 8,
c is 2 to 100,
R¹ and R² , independently of one another, are C₁-C₈-alkyl, benzyl or phenyl,
Z¹ and Z² independently of one another, are OH, NHR³ or a radical of the formula II
- (CH₂CH₂O)ᵥ(CH₂CH(CH₃)O)_{w}-H (II),
where
in the formula II the order of the alkylene oxide units is arbitrary and
v and w, independently of one another, are an integer from 0 to 200, where the sum of v and w is > 0,
R³ is hydrogen, C₁-C₈-alkyl or C₅-C₈-cycloalkyl;
- polysiloxanes of the formula I.2 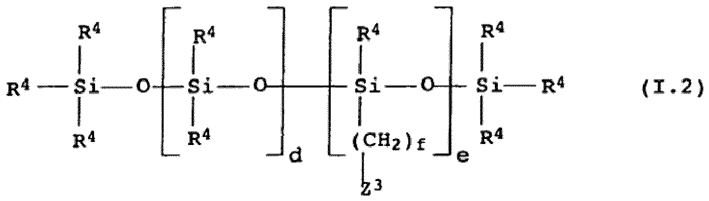 in which
the order of the siloxane units is arbitrary,
the radicals R⁴ are each, independently of one another, C₁-C₈-alkyl, preferably methyl, benzyl or phenyl,
d is an integer from 5 to 1000,
e is an integer from 2 to 100,
f is an integer from 2 to 8,
Z³ is OH, NHR³, where R³ is as defined above, or
a radical of the formula III
- (OCH₂CH₂) ₓ(OCH (CH₃) CH₂)_{y} -OH
where
in the formula III the order of the alkylene oxide units is arbitrary,
x and y, independently of one another, are an integer from 0 to 200, where the sum of x and y is > 0,
and mixtures thereof.

3. A water-soluble or water-dispersible polymer which comprises, in copolymerized form, at least one polyether-urethane as defined in either of claims 1 and 2, and at least one free-radically polymerizable compound which has at least one a,b-ethylenically unsaturated double bond.

4. The polymer according to claim 3, which comprises, in copolymerized form, at least one free-radically polymerizable hydrophilic nonionic compound M1).

5. The polymer according to claim 4, where the compound M1) is chosen from primary amides of a,b-ethylenically unsaturated monocarboxylic acids, N-vinyllactams, N-vinylamides of saturated monocarboxylic acids, esters of a,b-ethylenically unsaturated mono- and dicarboxylic acids with C₂-C₄-alkane- diols, amides of a,b-ethylenically unsaturated mono- and dicarboxylic acids with C₂-C₄-aminoalcohols which have a primary or secondary amino group, vinyl ethers, nonionic, hydrophilic vinyl- and allyl-substituted heterocyclic compounds and mixtures thereof.

6. The polymer according to claim 5, which comprises, in copolymerized form, a compound M1) chosen from acrylamide, methacrylamide, N-vinylpyrrolidone, N-vinylcaprolactam, N-vinylformamide, N-vinylacetamide and mixtures thereof.

7. The polymer according to any of claims 4 to 6, which additionally comprises, in copolymerized form, at least one free-radically polymerizable compound M2) with an a,b-ethylenically unsaturated double bond and at least one ionogenic and/or ionic group per molecule.

8. The polymer according to any of claims 4 to 7, which additionally comprises, in copolymerized form, at least one free-radically polymerizable crosslinking compound with at least two a,b-ethylenically unsaturated double bonds per molecule.

9. The polymer according to any of claims 4 to 8, which is obtainable by free-radical copolymerization in the presence of a component d) chosen from
d1) polyether-containing compounds,
d2) polymers which have at least 50% by weight of repeat units derived from vinyl alcohol,
d3) starch and starch derivatives,
and mixtures thereof.

10. The polymer according to any of claims 4 to 9, obtainable by free-radical polymerization of
- 1 to 25% by weight, based on the total weight of the components used for the polymerization, of at least one polyallyl-polyether-urethane,
- 50 to 99% by weight of at least one free-radically polymerizable nonionic compound M1),
- 0 to 25% by weight of at least one monomer M2) with at least one ionogenic and/or ionic group per molecule,
- 0 to 10% by weight of at least one crosslinker,
optionally in the presence of up to 25% by weight of at least one component d), as defined in claim 9.

11. A process for the preparation of a polymer as defined in any of claims 4 to 10 by free-radical polymerization in an aqueous solvent at a pH of from 5.5 to 8.0.

12. The process according to claim 11, comprising a first polymerization step and a subsequent second polymerization step, where the reaction mixture between the first and second polymerization step is subjected to stripping with steam or to a steam distillation.

13. A cosmetic or pharmaceutical composition comprising
A) at least one water-soluble or water-dispersible polymer as defined in any of claims 3 to 10, and
B) at least one cosmetically acceptable carrier.

14. The composition according to claim 13, where component B) is chosen from
i) water,
ii) water-miscible organic solvents, preferably C₁-C₄-alkanols,
iii) oils, fats, waxes,
iv) esters of C₆-C₃₀-monocarboxylic acids with mono- , di- or trihydric alcohols which are different from iii),
v) saturated acyclic and cyclic hydrocarbons,
vi) fatty acids,
vii) fatty alcohols
and mixtures thereof.

15. The composition according to claim 13 or 14, further comprising at least one constituent different from copolymer A which is chosen from cosmetically active ingredients, emulsifiers, surfactants, preservatives, perfume oils, thickeners, hair polymers, hair and skin conditioners, graft polymers, water-soluble or dispersible silicone-containing polymers, light protection agents, bleaches, gel formers, care agents, colorants, tints, tanning agents, dyes, pigments, bodying agents, humectants, refatting agents, collagen, protein hydrolysates, lipids, antioxidants, antifoams, antistats, emollients and softeners.

16. The composition according to any of claims 13 to 15 in the form of a solution, a gel, wax, foam, spray, an ointment, cream, emulsion, suspension, lotion, milk or paste.

17. The use of a polymer as defined in any of claims 3 to 10 in skin cleansing compositions, compositions for the care and protection of the skin, nail care compositions, preparations for decorative cosmetics and hair treatment compositions.

18. The use according to claim 17 in hair treatment compositions as thickener, setting agent and/or as conditioner.

19. The use according to claim 18, where the composition is in the form of a hair gel, hair mousse, shampoo, setting foam, hair tonic, hair spray or hair foam.

20. The use of a polymer as defined in any of claims 3 to 10 as auxiliary in pharmacy preferably as or in (a) coating composition(s) for solid medicament forms, and as or in (a) coating composition(s) for the textile, paper, printing and leather industry, and also for agrochemistry.

## Revendications

1. Polyéther-uréthane, contenant au moins un groupe allyle, qui contient incorporés
a) au moins un polyéther, qui contient un groupe réactif avec les groupes isocyanate et un groupe allyle,
b) au moins un composé qui contient au moins deux groupes réactifs avec les groupes isocyanate, choisi parmi les composés ayant un poids moléculaire moyen en nombre supérieur à 280, qui contiennent au moins deux atomes d'hydrogène actifs et au moins un groupe siloxane par molécule, et
c) au moins un polyisocyanate.

2. Polyéther-uréthane selon la revendication 1, qui contient incorporé au moins un composé choisi parmi :
- les polysiloxanes de formule générale I.1 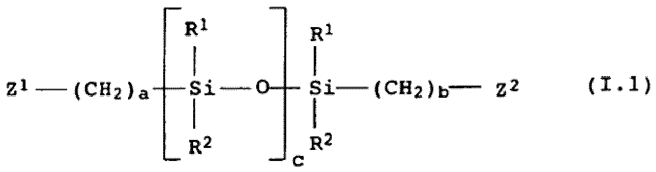 dans laquelle
a et b représentent indépendamment l'un de l'autre 1 à 8,
c représente 2 à 100,
R¹ et R² représentent indépendamment l'un de l'autre alkyle en C₁-C₈, benzyle ou phényle,
Z¹ et Z² représentent indépendamment l'un de l'autre OH, NHR³ ou un radical de formule II
-(CH₂CH₂O)ᵥ(CH₂CH(CH₃)O)_{w}-H (II)
l'ordre des unités oxyde d'alkylène dans la formule II étant quelconque et
v et w représentant indépendamment l'un de l'autre un nombre entier de 0 à 200, la somme de v et w étant > 0,
R³ représente hydrogène, alkyle en C₁-C₈ ou cycloalkyle en C₅-C₈ ;
- les polysiloxanes de formule générale I.2 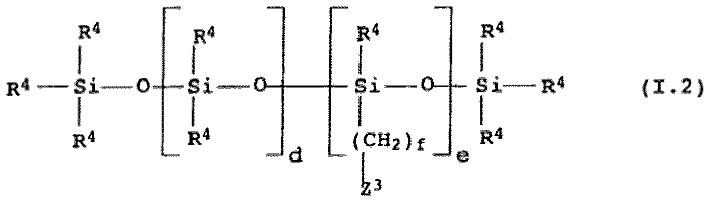 dans laquelle
l'ordre des unités siloxane est quelconque,
les radicaux R⁴ représentent à chaque fois indépendamment les uns des autres alkyle en C₁-C₈, de préférence méthyle, benzyle ou phényle,
d représente un nombre entier de 5 à 1 000,
e représente un nombre entier de 2 à 100,
f représente un nombre entier de 2 à 8,
Z³ représente OH, NHR³, R³ étant tel que défini précédemment, ou un radical de formule III
- (OCH₂CH₂) ₓ(OCH (CH₃) CH₂) _{y}-OH
l'ordre des unités oxyde d'alkylène dans la formule III étant quelconque,
x et y représentant indépendamment l'un de l'autre un nombre entier de 0 à 200, la somme de x et y étant > 0,
et leurs mélanges.

3. Polymère soluble dans l'eau ou dispersible dans l'eau, qui contient sous forme copolymérisée au moins un polyéther-uréthane tel que défini dans l'une quelconque des revendications 1 ou 2, et au moins un composé polymérisable par voie radicalaire, qui comprend au moins une double liaison α,β-éthyléniquement insaturée.

4. Polymère selon la revendication 3, qui contient sous forme copolymérisée au moins un composé hydrophile non ionique polymérisable par voie radicalaire M1).

5. Polymère selon la revendication 4, dans lequel le composé M1) est choisi parmi les amides primaires d'acides monocarboxyliques α,β-éthyléniquement insaturés, les N-vinyllactames, les N-vinylamides d'acides monocarboxyliques saturés, les esters d'acides mono- et dicarboxyliques α,β-éthyléniquement insaturés avec des alcane-diols en C₂-C₄, les amides d'acides mono- et dicarboxyliques α,β-éthyléniquement insaturés avec des amino-alcools en C₂-C₄ qui comprennent un groupe amino primaire ou secondaire, les éthers de vinyle, les composés hétérocycliques hydrophiles non ioniques à substitution vinyle et allyle, et leurs mélanges.

6. Polymère selon la revendication 5, qui contient sous forme copolymérisée un composé M1) qui est choisi parmi l'amide de l'acide acrylique, l'amide de l'acide méthacrylique, la N-vinylpyrrolidone, le N-vinylcaprolactame, le N-vinylformamide, le N-vinylacétamide et leurs mélanges.

7. Polymère selon l'une quelconque des revendications 4 à 6, qui contient également sous forme copolymérisée au moins un composé M2) polymérisable par voie radicalaire comprenant une double liaison α,β-éthyléniquement insaturée et au moins un groupe ionogène et/ou ionique par molécule.

8. Polymère selon l'une quelconque des revendications 4 à 7, qui contient également sous forme copolymérisée au moins un composé réticulant polymérisable par voie radicalaire comprenant au moins deux doubles liaisons α,β-éthyléniquement insaturées par molécule.

9. Polymère selon l'une quelconque des revendications 4 à 8, qui peut être obtenu par copolymérisation radicalaire en présence d'un composant d), qui est choisi parmi
d1) les composés contenant un polyéther,
d2) les polymères qui comprennent au moins 50 % en poids d'unités de répétition dérivant d'alcool vinylique,
d3) l'amidon et les dérivés d'amidon,
et leurs mélanges.

10. Polymère selon l'une quelconque des revendications 4 à 9, pouvant être obtenu par polymérisation radicalaire de
- 1 à 25 % en poids, par rapport au poids total des composants utilisés pour la polymérisation, d'au moins un polyallylpolyéther-uréthane,
- 50 à 99 % en poids d'au moins un composé non ionique polymérisable par voie radicalaire M1),
- 0 à 25 % en poids d'au moins un monomère M2) comprenant au moins un groupe ionogène et/ou ionique par molécule,
- 0 à 10 % en poids d'au moins un agent de réticulation,
éventuellement en présence de jusqu'à 25 % en poids d'au moins un composant d) tel que défini dans la revendication 9.

11. Procédé de fabrication d'un polymère tel que défini dans l'une quelconque des revendications 4 à 10, par polymérisation radicalaire dans un solvant aqueux à un pH de 5,5 à 8,0.

12. Procédé selon la revendication 11, comprenant une première étape de polymérisation et une seconde étape de polymérisation ultérieure, le mélange réactionnel étant soumis à une extraction avec de la vapeur d'eau ou à une distillation à la vapeur d'eau entre la première et la seconde étape de polymérisation.

13. Agent cosmétique ou pharmaceutique, contenant
A) au moins un polymère soluble dans l'eau ou dispersible dans l'eau tel que défini dans l'une quelconque des revendications 3 à 10 et
B) au moins un véhicule cosmétiquement acceptable.

14. Agent selon la revendication 13, dans lequel le composant B) est choisi parmi
i) l'eau,
ii) les solvants organiques miscibles avec l'eau, de préférence les alcanols en C₁-C₄,
iii) les huiles, les graisses, les cires,
iv) les esters différents de iii) d'acides monocarboxyliques en C₆-C₃₀ avec des alcools mono-, bi- ou trivalents,
v) les hydrocarbures acycliques et cycliques saturés,
vi) les acides gras,
vii) les alcools gras,
et leurs mélanges.

15. Agent selon l'une quelconque des revendications 13 ou 14, contenant également au moins un constituant différent du copolymère A, qui est choisi parmi les agents actifs cosmétiques, les émulsifiants, les tensioactifs, les conservateurs, les huiles parfumées, les épaississants, les polymères pour cheveux, les revitalisants capillaires et cutanés, les polymères greffés, les polymères contenant de la silicone solubles ou dispersibles dans l'eau, les agents photoprotecteurs, les agents blanchissants, les agents gélifiants, les agents de soin, les agents de coloration, les teintures, les agents de brunissement, les colorants, les pigments, les régulateurs de consistance, les agents de rétention de l'humidité, les agents hydratants, le collagène, les hydrolysats de blanc d'oeuf, les lipides, les antioxydants, les démoussants, les antistatiques, les émollients et les plastifiants.

16. Agent selon l'une quelconque des revendications 13 à 15 sous la forme d'une solution, d'un gel, d'une cire, d'une mousse, d'un spray, d'un onguent, d'une crème, d'une émulsion, d'une suspension, d'une lotion, d'un lait ou d'une pâte.

17. Utilisation d'un polymère tel que défini dans l'une quelconque des revendications 3 à 10 dans des agents de nettoyage de la peau, des agents de soin et de protection de la peau, des agents de soin des ongles, des préparations de cosmétique décorative et des agents de traitement des cheveux.

18. Utilisation selon la revendication 17 dans des agents de traitement des cheveux en tant qu'épaississant, fixateur et/ou en tant que revitalisant.

19. Utilisation selon la revendication 18, dans laquelle l'agent se présente sous la forme d'un gel pour les cheveux, d'une mousse pour les cheveux, d'un shampoing, d'une mousse de fixation, d'une cire pour les cheveux, d'un spray pour les cheveux ou d'une mousse pour les cheveux.

20. Utilisation d'un polymère tel que défini dans l'une quelconque des revendications 3 à 10, en tant qu'adjuvant en pharmacie, de préférence en tant que ou dans un ou plusieurs agents de revêtement pour formes pharmaceutiques solides, ainsi qu'en tant que ou dans un ou plusieurs agents de revêtement pour l'industrie du textile, du papier, de l'impression et du cuir, ainsi que pour l'agrochimie.
